Europäisches Patentamt

European Patent Office

Office européen des brevets

(19)

(11) Publication number: **0 166 996 B2**

(12)

# NEW EUROPEAN PATENT SPECIFICATION

(45) Date of publication of the new patent specification: **21.12.94**

(51) Int. Cl.5: **C12N 15/28**, C12P 21/02, C07K 13/00, C12P 19/34

(21) Application number: **85106915.3**

(22) Date of filing: **04.06.85**

The file contains technical information submitted after the application was filed and not included in this specification

(54) **A method for stabilizing a physiologically active substance produced by recombinant DNA technique and a stable aqueous solution or powder containing the same.**

(30) Priority: **07.06.84 JP 115496/84**

(43) Date of publication of application:
**08.01.86 Bulletin 86/02**

(45) Publication of the grant of the patent:
**16.01.91 Bulletin 91/03**

(45) Mention of the opposition decision:
**21.12.94 Bulletin 94/51**

(84) Designated Contracting States:
**BE CH DE FR GB IT LI NL SE**

(56) References cited:
EP-A- 0 155 549      DE-A- 3 019 612
DE-A- 3 040 005      JP-A-81 106 991
JP-A-81 108 020      JP-A-81 115 723
JP-A-81 126 422      JP-A-83 013 521
JP-A-83 065 218      JP-B- 118 718
US-A- 3 637 640      US-A- 4 447 355

THE BRITISH JOURNAL OF CANCER, vol. 44, no. 3, September 1981, London; N. MAT-THEWS: "Tumor-Necrosis Factor from the Rabbit. V. Synthesis in Vitro by Mononuclear Phagocytes from Various Tissues of Normal and BCG-Injected Rabbits", pp. 418-424

(73) Proprietor: **Asahi Kasei Kogyo Kabushiki Kaisha**
**2-6, Dojimahama 1-chome**
**Kita-ku**
**Osaka-shi**
**Osaka 530 (JP)**

(72) Inventor: **Sakamoto, Hajimu**
**3984-19 Imaizumi**
**Fuji-shi**
**Shizuoka-ken (JP)**
Inventor: **Kiyota, Takao**
**125-8 Mitojima**
**Fuji-shi**
**Shizuoka-ken (JP)**
Inventor: **Itoh, Hirataka**
**490-341 Nakano**
**Fuji-shi**
**Shizuoka-ken (JP)**
Inventor: **Hayashi, Hiroshi**
**3989-1 Imaizumi**
**Fuji-shi**
**Shizuoka-ken (JP)**

EP 0 166 996 B2

Rank Xerox (UK) Business Services
(3. 10/3.09/3.3.3)

Matthews, 1981, Immunology, vol. 44, pp. 135-142

Aggrawal et al., 1985, Journal Biol. Chemistry, vol. 260, pp. 2345-2354

Pennica et al., 1984, Nature, vol. 312, pp. 724-729

Shiria et al., 1985, Nature, vol. 313, pp. 803-806

Ito et al., 1986, DNA, vol. 5, pp. 157-165

Hsu et al., 1982, CA, vol.96, no. 195772r

Sokolovskii, 1981, CA, vol. 94, no. 36274x

Kauffmann et al., 1980, CA, vol.92, no.56624y

Berezin, 1979, CA, vol.90, no.116804c

Mitusi & Mizuno, 1970, CA, vol. 72, no.18529a

Bich & Fekete, 1977, CA, vol. 87, no.44226d

Norman et al., CA, 1979, CA, vol.90, no.37550p

Fojo et al., 1977, Biochim. Biophys. Acta, vol. 494. pp. 92-99

Sedmak et al., 1977, Methods Enzymol., vol. 78, pp. 591-595

Papermaster & Baron, 1982, Texas Reports on Biology & Medicine, vol. 41, pp. 672-680

(74) Representative: **Boeters, Hans Dietrich, Dr. et al**
**Patentanwälte Boeters & Bauer**
**Bereiteranger 15**
**D-81541 München (DE)**

**Description**

This invention relates to a specific physiologically active substance produced by recombinant DNA technique, and more particularly to a stable aqueous solution of powder containing this physiologically active substance and an effective amount of an albumin. This invention also relates to a method for stabilizing the said physiologically active substance which comprises adding an albumin to an aqueous solution or powder containing the physiologically active substance which is prepared by recombinant DNA technique and has an antitumor activity.

It is known that there is a physiologically active substance which is derived from human and which has cytotoxic activity against L-M cells and is capable of inducing hemorrhagic necrosis of a transplanted tumor, Meth A sacroma, in the BALB/c mouse. Such a physiologically active substance has an antitumor activity and is called human Tumor Necrosis Factor (hereinafter often referred to as "human TNF"). It is also known that such a physiologically active substance has no toxic effect upon normal cells in vitro. Further, since the physiologically active substance is derived from human, there is no danger that anaphylactic shock might be caused due to the administration of a substance to a human which is not the origin of the substance. Therefore, expectations for the clinical application of the above-mentioned physiologically active substance as an antitumor medicine have been great in the art.

In order to ensure the wide and safe clinical application of human TNF as an antitumor medicine, it is required to obtain human TNF in highly purified form. Previously, there has been proposed a method for obtaining human TNF by recombinant DNA technique. According to the proposed method, the desired human TNF can be produced in substantially pure form on a large scale. The proposed method consists in:

ligating a deoxyribonucleic acid coding for human TNF to a replicable expression vehicle to obtain a replicable recombinant DNA comprising said deoxyribonucleic acid and said replicable expression vehicle,

transforming cells of a microorganism or cell culture with said replicable recombinant DNA to form transformants;

selecting said transformants from parent cells of the microorgranism or cell culture;

incubating said transformants, causing said transformants to express said deoxyribonucleic acid and produce human TNF; and

isolating said human TNF from the incubated transformants, followed by purification.

As stated above, according to the proposed method using recombinant DNA technique, there can advantageously be obtained the desired human TNF in substantially pure form on a large scale. However, when large-scale production of human TNF to be used as an antitumor medicine is performed, it is usually needed to store the substantially pure human TNF in the form of a solution or a frozen mass over a prolonged period of time and lyophilize EP the active substance solution. However, the present inventors have found that the activity of substantially pure human TNF markedly drops on storing, freezing, thawing and lyophilizing it. Further, it is noted that during the storage of the substantially pure TNF produced by recombinant DNA technique until the TNF is actually administered to a patient suffering from cancer, the activity of the TNF also drops markedly. With respect to the stabilization of proteins having antitumor activity, there are publications such as USP 4,447,355 and Japanese Patent Application Laid-Open Specification Nos. 59-39829 (1984) and 59-59625 (1984). However, as far as the present inventors are aware, there has been no report in which the stability of substantially pure human TNF produced by recombinant DNA technique is studied. Under these circumstances, the efficient and steady supply of the substantially pure human TNF, especially on a commercial scale cannot be ensured, despite the knowledge that the human TNF is an effective antitumor medicine.

To overcome the above-elucidated difficulty with respect to the stability of the physiologically active substance, the present inventors have made extensive and intensive studies. As a result, it has been found, quite surprisingly, that there can be provided a stable aqueous solution or powder, comprising an effective amount of an albumin as a stabilizing agent and the physiologically active substance which substance may be stored over a prolonged period of time without losing its activity and has been rendered stable during the procedures of isolation and purification and on freezing, thawing, lyophilization or the like. Based on the novel finding, the present invention has been completed.

It is therefore an object of the present invention to provide a stable aqueous solution or powder of a specific physiologically active substance which maintains its activity over a prolonged period of time and which is stable on freezing, thawing, lyophilization or the like.

It is, another object of the present invention to provide a method for stabilizing the said physiologically active substance produced by recombinant DNA technique.

The foregoing and other objects, features and advantages of the present invention will be apparent to those skilled in the art from the following detailed description and appended claims taken in connection with

EP 0 166 996 B2

the accompanying drawings in which:

Fig. 1 illustrates the restriction maps of plasmid inserts each containing a DNA coding for a conventional rabbit physiologically active polypeptide;

Fig. 2 illustrates the flow-sheet of the method for the preparation of a recombinant DNA (pTNF-lac-1) coding for the conventional rabbit physiologically active polypeptide;

Fig. 3 illustrates the flow-sheet of the method for the preparation of another recombinant DNA (pTNF-lacUV5-1) coding for the conventional rabbit physiologically active polypeptide;

Fig. 4 illustrates the restriction map of the portion of a plasmid containing a gene for a physiologically active substance to be used in the present invention,

Fig. 5 illustrates the restriction map of the portion of a plasmid containing a gene for a conventional rabbit TNF;

Fig. 6 illustrates the flow-sheet of the method for the preparation of a recombinant DNA (pHTNF-lacUV5-1) coding for a physiologically active substance to be used in the present invention;

Fig. 7 illustrates the flow-sheet of the method for the preparation of another recombinant DNA (pHTNF-lacUV5-2) coding for the physiologically active substance to be used in the present invention; and

Fig. 8 illustrates the graph showing the effect of the concentration of human serum albumin on the remaining activity of the physiologically active substance to be used in the present invention after storage at 4°C for 7 days.

In one aspect of the present invention, there is provided a stable aqueous solution or powder containing a physiologically active substance and an effective amount of an albumin, said physiologically active substance being a specific one which is produced by recombinant DNA technique using a recombinant DNA containing a DNA coding for the physiologically active substance, and which has cytotoxic activity against L-M cells and is capable of inducing hemorrhagic necrosis of transplanted Meth A sercoma in the BALC/c mouse.

In another aspect of the present invention, there is provided a method for stabilizing a physiologically active substance, which comprises adding to an aqueous solution or powder containing, a physiologically active substance an effective amount of an albumin.

said physiologically active substance being a specific one which is produced by recombinant DNA technique using a recombinant DNA containing a DNA coding for the physiologically active substance, and which has cytotoxic activity against L-M cells and is capable of inducing hemorrhagic necrosis of transplanted Meth A sarcoma in the BALB/c mouse.

As mentioned before, the specific physiologically active substance to be used in the present invention is one produced by a customary recombinant DNA technique using a recombinant DNA containing a DNA coding for the said physiologically active substance. When the physiologically active substance is subjected to assays according to the methods described later, the physiologically active substance exhibits cytotoxic activity against L-M cells and induces hemorrhagic necrosis of transplanted Meth A sarcoma in the BALB/c mouse. Specifically, the said physiologically active substance is a polypeptide having an amino acid sequence represented by the following formula (I):

4

```
Ser Ser Ser Arg Thr Pro Ser Asp Lys Pro Val Ala His Val Val

Ala Asn Pro Gln Ala Glu Gly Gln Leu Gln Trp Leu Asn Arg Arg

Ala Asn Ala Leu Leu Ala Asn Gly Val Glu Leu Arg Asp Asn Gln

Leu Val Val Pro Ser Glu Gly Leu Tyr Leu Ile Tyr Ser Gln Val

Leu Phe Lys Gly Gln Gly Cys Pro Ser Thr His Val Leu Leu Thr

His Thr Ile Ser Arg Ile Ala Val Ser Tyr Gln Thr Lys Val Asn

Leu Leu Ser Ala Ile Lys Ser Pro Cys Gln Arg Glu Thr Pro Glu

Gly Ala Glu Ala Lys Pro Trp Tyr Glu Pro Ile Tyr Leu Gly Gly

Val Phe Gln Leu Glu Lys Gly Asp Arg Leu Ser Ala Glu Ile Asn

Arg Pro Asp Tyr Leu Asp Phe Ala Glu Ser Gly Gln Val Tyr Phe

Gly Ile Ile Ala Leu
```

wherein Gln stands for a glutamine residue, Asp as aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tryptophan residue, Arg an arginine residue, Met a methionine residue, and Cys a cysteine residue.

The physiologically active substance having the above-mentioned amino acid sequence may be produced by a customary recombinant DNA technique using a recombinant DNA which contains a DNA coding for the physiologically active substance comprising at least one base sequence selected from the group consisting of a base sequence represented by the following formula (II) and a complementary base sequence to said base sequence:

```
TCA TCT TCT CGA ACC CCG AGT GAC AAG CCT GTA GCC CAT GTT GTA

GCA AAC CCT CAA GCT GAG GGG CAG CTC CAG TGG CTG AAC CGC CGG

GCC AAT GCC CTC CTG GCC AAT GGC GTG GAG CTG AGA GAT AAC CAG

CTG GTG GTG CCA TCA GAG GGC CTG TAC CTC ATC TAC TCC CAG GTC

CTC TTC AAG GGC CAA GGC TGC CCC TCC ACC CAT GTG CTC CTC ACC

CAC ACC ATC AGC CGC ATC GCC GTC TCC TAC CAG ACC AAG GTC AAC

CTC CTC TCT GCC ATC AAG AGC CCC TGC CAG AGG GAG ACC CCA GAG

GGG GCT GAG GCC AAG CCC TGG TAT GAG CCC ATC TAT CTG GGA GGG

GTC TTC CAG CTG GAG AAG GGT GAC CGA CTC AGC GCT GAG ATC AAT

CGG CCC GAC TAT CTC GAC TTT GCC GAG TCT GGG CAG GTC TAC TTT

GGG ATC ATT GCC CTG
```

wherein A stands for a deoxyadenylic acid residue, G a deoxyguanylic acid residue, C a deoxycytidylic acid residue and T thymidylic acid residue and wherein the left end and right end of the formula (II) represent 5'-hydroxyl group side and 3'-hydroxyl group side, respectively.

The above-mentioned physiologically active substance and the above mentioned DNA may be obtained as follows:

5

1. A bacteriophage λ/rabbit genomic library and a bacteriophage λ/human genomic library prepared by Prof. T. Maniatis, Department of Biochemistry and Molecular Biology, Harvard University, 7 Divinity Avenue, Cambridge, Massachusetts 02138, U.S.A. are used. These materials may be prepared according to the following procedures [see Cell, *15,* p. 687 (1978)]:

(1) rabbit or human tissues, for example rabbit or human pancreas tissue, are reduced to frozen powder and treated to digest RNA and protein materials and provide, on precipitation, high molecular weight rabbit or human DNA;

(2) the high molecular weight DNA is partially digested for random cutting with respect to gene locus;

(3) the resultant DNA fragments are size-fractionated giving from 15 to 20 kilo base pair (kb) fragments;

(4) the resultant fragments of Step 3 are cloned using a λ Charon 4A phage vector; and

(5) the resultant vectors are packaged in vitro to infectious phage particles containing rDNA to obtain the above-mentioned rabbit or human genomic library.

2. The rabbit TNF cDNA obtained in Referential Example 1 is $^{32}$P-labelled by P. W. J. Rigby et al's nick translation method [see J. Mol. Biol. *113*, p.237 (1977)].

3. Each of the bacteriophage λ/rabbit genomic library and bacteriophage λ/human genomic library is plated to virtual confluence on a lawn of bacteria and screened from hybridization with the $^{32}$P-labelled rabbit TNF cDNA.

4. From the appropriate clones, the corresponding DNA is isolated, restriction mapped and analyzed by Southern hybridization [see E. M. Southern, J. Mol. Biol., *98*, p.503 (1975)]. Restriction fragments containing rabbit or human TNF genes are subcloned into plasmid vectors and then sequenced.

5. The base sequence of the rabbit TNF cDNA is compared with that of the rabbit TNF gene to determine the exons (certain sequences of bases which code for the amino acid sequence of rabbit TNF) and introns (certain sequences of bases which do not code for the amino acid sequence of rabbit TNF) of the rabbit TNF gene.

6. Thereafter, the base sequence of the human TNF gene is compared with that of the rabbit TNF gene to determine the exons and introns of the human TNF gene.

7. The amino acid sequence of rabbit TNF that has been deduced from the base sequence obtained by deleting the introns of the rabbit TNF gene and combining the exons thereof is affirmed to be in agreement with that deduced from the base sequence of the rabbit TNF cDNA.

8. Next, the amino acid sequence of the physiologically active substance to be used in the present invention is deduced from the base sequence of the DNA coding for the physiologically active substance obtained by deleting the introns of the gene coding for the physiologically active substance and combining the exons thereof. The amino acid sequence of the physiologically active substance is affirmed to be partially in agreement with that of the rabbit TNF.

9. Then, the DNA coding for the physiologically active substance is tailored into vitro for insertion into an appropriate expression vehicle to form recombinant DNA containing the coding DNA. The recombinant DNA is used to transform an appropriate host cell which is, in turn, permitted to grow in a culture and to express the desired physiologically active substance.

10. The physiologically active substance thus produced has 155 amino acid residues in its mature form, beginning with serine. When it has a signal peptide in its presequence, the signal peptide is very hydrophobic in character.

The foregoing discloses the procedures for obtaining the gene coding for the physiologically active substance, the base sequence of the DNA coding for the physiologically active substance and the process for producing the physiologically active substance by the use of the DNA. However, it will be understood that the foregoing disclosure is not intended to limit the method and that obvious changes may be made by those skilled in the art.

Due to the variable use frequency of a codon (genetic code) corresponding to each amino acid and for other reasons, a partial or entire portion of the base sequence of the DNA coding for the physiologically active substance may be substituted by an organo-chemically synthesized artificial DNA without causing the amino acid sequence of the polypeptide obtained therefrom to be changed.

Presumably, the physiologically active substance may be intracellularly produced in immature form as a prepeptide or prepropeptide, which may be processed via an intermediate form to a mature physiologically active substance in the processing stage. The immature form of the physiologically active substance may be deduced from the base sequence of the human TNF gene. The DNA comprising a DNA encoding the physiologically active substance in immature or intermediate form may also be recombined with a natural or artificially synthesized DNA.

EP 0 166 996 B2

One application of this technique may be attained by inserting the methionine codon (ATG) in the 5'-end and inserting at least one stop codon selected from TAA, TAG and TGA in the 3'-end of the mature or intermediate or immature TNF DNA. Due to the presence of the methionine coden, the mature or intermediate or immature physiologically active substance may be produced on the mRNA synthesized with the aid of an appropriate promotor. However, the methionine residue attached to the N-terminus of the physiologically active substance is cleaved or not cleaved according to the kind of the host cell employed. The purpose of inserting the stop codon is to stop translation of the mRNA transcribed from the DNA coding for the physiologically active substance at an appropriate position (C-terminus of polypeptide of the formula I).

Another application of this technique may be attained by adding to the DNA a higly hydrophobic base sequence known as a "signal sequence". By this addition, it may become feasible to secrete the physiologically active substance to outside the host cell or, in the case of a gram-negative bacterium, into the space known as "periplasm".

When a vector in which a start codon is incorporated is employed, a fused peptide may be produced which consists of the physiologically active substance and a peptide attributed to the vector. In this case, the fused peptide may be cleaved chemically or enzymatically. Alternatively, the fused peptide, if the main activity of the physiologically active substance is not adversely affected, may be used as it is.

The DNA coding for the physiologically active substance may be connected, at its region upstream of the 5'-end, to the gene sequence of a promoter thereby to obtain a TNF DNA-promoter sequence which does not hinder its replication and does not cause translation of the resultant RNA to be adversely affected. The thus obtained TNF DNA-promoter sequence may be combined with a vector which is replicable in a bacterium or higher organism cell to obtain a recombinant DNA. The thus obtained recombinant DNA may be used to transform a bacterium or higher organism cell used as a host. The thus obtained transformant may be cultured to effect expression of the DNA coding for the physiologically active substance in order to produce the physiologically active substance.

When *Escherichia coli* is used as the above-mentioned host, there may be mentioned, as the suitable host, various mutant strains of *E. coli* K-12, such as HB101 (ATCC 33694), C600K (ATCC 33955), D1210, RRI (ATCC 31343), MC1061, LE392 (ATCC 33572), JM101 (ATCC 33876), JM83, and $\chi$1776 (ATCC 31244). When the *E. coli* host is employed, there may be mentioned, as the suitable vector, plasmids such as pBR322, pBR325, pBR327, pUC3, pUC9, pMB9 (ATCC 37019), pJB8 (ATCC 37074) and pKC7 (ATCC 37084), $\lambda$ phages such as $\lambda$gt, $\lambda$B and Charon 4A, and M13 phage. To have the active substance produced in the *E. coli* cell, a promoter selected from the promoters of the *E. coli* and phage genes may be employed. Examples of the suitable promoter include the genes for lactose degradation enzyme (LAC), UV5 mutant thereof, penicillinase (BLA) and tryptophan synthetase (TRP), $\lambda$ phage $P_L$ promoter and *tac* promoter which is a fused promoter of tryptophan synthetase and lactose degradation enzyme.

When *Bacillus subtilis* is used as the host, there may be mentioned, as the suitable host, BD170 strain (ATCC 33608), BR151 strain (ATCC 33677) and MI112 strain (ATCC 33712). When the *Bacillus subtilis* host is employed, there may be mentioned, as the suitable vector, plasmids pC194 (ATCC 37034), pUB110 (ATCC 37015), pSA2100 (ATCC 37014) and pE194. Further, when the *Bacillus substilis* host is employed, there may be mentioned, as the suitable promoter, the genes for chloramphenicol acetylation enzyme (CAT), penicillinase and anti-erythromycin.

When a yeast is used as the host, there may be mentioned, as the suitable host, strains of *Saccharomyces cerevisiae* such as RH218 (ATCC 44076), SHY1 (ATCC 44769), SHY3 (ATCC 44771), D131A, 483 and 830. When the yeast host is employed, there may be mentioned, as the suitable vector, plasmids such as YEp13 (ATCC 37115), YEp6, YRp7 and YIp5. Further, when the yeast host is employed, there may be mentioned, as the suitable promoter, the genes for acid phosphatase, alcohol dehydrogenase (ADHI), tryptophan synthetase (TRP), phosphoglycerate kinase (PGK), cytochrome B (COB) and actin.

The thus prepared transformant is cultured on a large scale according to a customary method to produce the desired physiologically active substance. Then, the supernatant of the culture or a cell extract is collected to obtain a crude physiologically active substance.

The crude physiologically active substance produced by any of the methods as set forth above may be purified using the below-cited conventional biochemical techniques singly or in combination to give an aqueous purified physiologically active substance solution, which is lyophilized to give a purified physiologically active substance powder. As the suitable biochemical technique for purification of the active substance, there can be mentioned, for example, a salting-out technique in which ammonium sulfate is employed, an ion exchange chromatography in which an anion exchange resin is employed, a gel filtration technique and an electrophoresis technique. As the purity of the active substance is increased by practicing the above techniques for purification, it is recognized that the active substance gradually becomes instable.

7

For example, a sample of the physiologically active substance so purified as to have a specific activity of 500,000 units/mg (the specific activity is expressed as units of activity per mg of protein; the unit of activity is defined later) is quite instable as seen from the data given in the Examples. Even the samples having a specific activity lower than 500,000 units/mg also experience a decrease of the respective activity in some degree when they are in storage or subjected to freezing, thawing, lyophilization and other operations.

Accordingly, the present invention is directed to the stabilization of the physiologically active substance that has been purified to a high degree and has been rendered instable. The active substance to be stabilized according to the present invention may be either in the form of a solution or powder. However, it is preferred that the active substance to be stabilized be in the form of a solution.

It is preferred that the solution of the physiologically active substance to be stabilized according to the present invention constantly have a pH value of from 5 to 10, and further, it is preferred that the solvent from the solution to be stabilized be a suitable buffer. As the suitable buffer, there can be mentioned, for example, a phosphate buffer and a tris (hydroxymethyl) aminomethane-HCl buffer. According to need, a salt, such as sodium chloride and potassium chloride, is added to the solution. For example, a salt is added to the solution so as to prepare an isotonic solution, when the solution is used for injection. The purpose of addition of a salt is not limited to the above. The concentration of such a salt in the solution, may be determined, depending on the purpose of addition of the salt. For example, when the ultimate TNF solution is used for injection, an isotonic solution is prepared from the solution by addition of sodium chloride up to a concentration of 0.15M.

According to the method of the present invention, an effective amount of an albumin is added to an aqueous solution or powder containing the physiologically active substance.

As the suitable albumin, there can be mentioned human albumin and bovine albumin. There may also be mentioned albumins from various animals, such as horse, sheep, goat and chicken. As specific examples of the suitable albumin, there can be mentioned bovine serum albumin, human serum albumin, bovine lactalbumin, human lactalbumin and albumin derived from human placenta. No significant difference is observed, in respect of the ability to stabilize the active substance, between the above-mentioned albumins. As the stabilizing agent for injection preparations, however, human serum albumin is most preferable. The above-mentioned albumins may be purified according to customary methods before use.

The albumin to be employed in the present invention is added to the solution in an amount of about 1 $\mu$g or more, preferably 10 $\mu$g or more, especially preferably 100 $\mu$g or more, per ml of the solution having a cytotoxic activity against L-M cells of $10^2$ to $10^9$ units/ml (the unit of activity is defined later). The upper limit of the amount of the albumin is usually determined from the viewpoints of the solubility of the albumin and viscosity of the resulting solution and from the economical viewpoint. The upper limit of the amount of albumin is generally 50 mg, preferably 10 mg, per ml of the TNF solution. When the active substance to be stabilized is in a powdery form, the albumin is added to the powder such an amount as will cause an aqueous solution, which is obtained by dissolving the powdery physiologically active substance to exhibit an activity of $10^2$ to $10^9$ units/ml, to have the above-mentioned concentrations of the albumin.

The way in which the albumin is added is not critical. For example, the albumin in a powdery form may be directly added to the active substance solution. Alternatively, the powder of the albumin may be dissolved, in advance, in water or a suitable buffer, and added to the solution. Further, alternatively, the powder of the albumin may be mixed with the powder of the active substance. Addition of the albumin may be effected at any time during the purification step or the step of manufacturing pharmaceutical preparations.

It is preferred that storing and purification of and manufacturing pharmaceutical preparations from the physiologically active substance solution in which an albumin to be employed according to the present invention is incorporated, if kept in the form of a solution, be performed at a temperature of from 0° to 30°C, more preferably from 0° to 10°C. When the solution is stored in a frozen form, it is preferred that the temperature for storage be maintained below 0°C, more preferably below -20°C.

The solution in which an effective amount of an albumin, according to the present invention, is incorporated does maintain its activity during the storing, whether it is in the form of a solution or in a frozen form, or during the steps of purification and manufacturing pharmaceutical preparations.

Further, the method for stabilizing the physiologically active substance of the present invention, is also applicable to lyophilization. Illustratively stated, when solutions of the physiologically active substance (especially, in the case of highly purified active substance) are subjected to lyophilization, the activities thereof generally markedly drop. However, the solutions containing an effective amount of an albumin, according to the present invention, are lyophilized without losing its activity to give a powder of the physiologically active substance. The powder may be dissolved to give a stable aqueous solution in which the concentrations of the albumin and the active substance fall within the range as defined above. The albumin may, alternatively be incorporatedc in the lyophilized preparations of the physiologically active substances. When the physiologically active substance is stored in a powdery form, it is preferred that the temperature for storage be maintained at room temperature or below.

In the below-mentioned in vivo assay in which a Meth A sarcoma transplanted mouse is used, when 300 units of the purified physiologically active substance which has been stabilized according to the present invention is administered to the mouse, the activity is evaluated as ( + ). Also, significant growth inhibition or regression of the cancer is observed after administration of the purified physiologically active substance, as compared with control mice which are injected with a physiological saline, in the mice which have been transplanted with colon carcinoma Colon 26. Further, the cytotoxic activity against various cancer cells of the purified physiologically active substance is evaluated in substantially the same manner as the in vitro assay method as mentioned later except that KB cells (adenocarcinoma), PC-8 cells (lung cancer), and normal cells (fetal human kidney cells and fetal human foreskin cells) as control are used instead of L-M cells and the cells are incubated at 37°C for 72 hours instead of at 37°C for 48 hours. As a result, the purified physiologically active substance exhibits significant cytotoxic activity against the cancer cells while exhibiting no cytotoxic activity against the normal cells. To assay the activity of TNF, there are usually employed two methods, i.e. the in vivo method in which the tumor necrosis effect is measured in vivo, and the in vitro method in which the cytotoxic effect on neoplastic cells is measured in vitro.

(1) In vivo assay method

As the in vivo method, there can be mentioned, for example, the method of Carswell et al [see Proc. Nat. Acad. Sci. USA, $72$(9), 3666-3670 (1975)]. According to this method, Meth A cells ($2 \times 10^5$ cells) are transplanted intradermally at armpit of each of BALB/c mice and, 7 days later, mice with tumors of 7-8 mm in diameter, good vascularization and no spontaneous central necrosis are selected for evaluation. A sample (0.5 ml) diluted with a physiological saline solution is injected through the tail vein of each of the mice. The activity of the sample is evaluated after 24 hours according to the following criterion.

(-):         no change
( + ):       slight hemorrhagic necrosis
( + + ):     moderate hemorrhagic necrosis (central necrosis extending over approximately 50% of the tumor surface)
( + + + ):   marked hemorrhagic necrosis (massive necrosis leaving a small viable rim along the tumor periphery)

(2) In vitro assay method

As the in vitro method for the assay of activity, there can be mentioned, for example, the method of Ruff et al [see Lymphokines, Vol. 2, edited by E. Pick, Academic Press, N.Y. 245-248 (1980)] and the method of Kull et al [see J. Immunol., $126$ (4), 1279-1283 (1981)].

The in vitro method that the present inventors have employed for the assay of activity has been developed by improving the above-mentioned conventional methods. The in vitro method of the present inventors, in which the cytotoxic activity of TNF against L-M cells (American Type Culture Collection CCL 1.2) is measured, is carried out as follows. As culture vessel, there are employed 96-well microtiter plates produced by Flow Laboratories, Inc. (U.S.A.), and L-M cells are cultured in Eagle's minimum essential medium [see Science, $130$, 432-437 (1959)] containing 10 v/v% fetal calf serum. A sample (0.1 ml) serially diluted with the medium and the L-M cell suspension (0.1 ml, $1 \times 10^4$ cells) are mixed in each well of the plates and the plates are incubated at 37°C for 48 hours in an air containing 5% carbon dioxide. At the end of the culture period, a 20% aqueous solution of glutaraldehyde (20 $\mu$l) is added to fix the cells. After fixation, the plates are washed with distilled water and allowed to dry, and 0.05% methylene blue (0.1 ml) is added to stain the viable cells. The plates are thoroughly washed with distilled water to remove excess dye and allowed to dry. 3% Hydrochloric acid (0.2 ml) is added to each well to extract the dye from stained cells. Absorbance of each well at 665 nm is measured with Titertek Multiskan produced by Flow Laboratories, Inc., USA. The absorbance is proportional to the number of viable cells. The activity, unit(U)-/ml, is defined as the reciprocal dilution of TNF that causes 50% cytotoxicity, and can be obtained by plotting the dilution versus the absorbance on a graph. All the activities, assayed according to the in vitro method, as used hereinafter are expressed by the above-defined unit except in Referential Examples 1 and 2. In Referential Examples 1 and 2, the activity is evaluated in substantially the same manner as described above except that L929 cells (American Type Culture Collection, CCL 1) are used instead of L-M cells and Eagle's minimum essential medium containing 1 v/v% fetal calf serum and 5 $\mu$g/ml actinomycin D is used

instead of Eagle's minimum essential medium containing 10 v/v% fetal calf serum, and that the incubation is effected for 21 hours instead of 48 hours.

According to the method of the present invention, efficient and steady supply, on a commercial scale, of the highly purified physiologically active substance, which is believed to be a clinically applicable effective antitumor medicine, can be ensured because in the method of the present invention, the activity of the active substance is maintained during the storing, whether the active substance is in the form of a solution, a frozen mass or a lyophilized preparation, and during the steps of purification and manufacturing pharmaceutical preparations. It has also been found that the solution or powder, in which human albumin is incorporated, can be safely administered to the human body, wherefore the novel composition of the present invention is especially useful when the physiologically active substance is clinically applied as an antitumor medicine.

The present invention will now be described in more detail with reference to the following Referential Example, Working Examples and Comparative Example that by no means limit the scope of the invention.

In practicing the present invention, construction of a recombinant DNA and insertion of a recombinant DNA to a microorganism were carried out in accordance with the procedure described in the following experimental reports (Literatures (1) to (4)], unless otherwise indicated.

(1) Yasutaka Takagi, Manual For Genetic Engineering, Kodan-sha, Tokyo.

(2) Yasuktaka Takagi, Experimental Method In Genetic Engineering, Kodan-sha, Tokyo.

(3) T. Maniatis, E. F. Fritsch, J. Sambrook, Molecular Cloning, Cold Spring Harbor Laboratory, New York.

(4) Ray Wu et al., Method in Enzymology, Vol. 101, Academic Press, New York.

Abbreviations used in Referential Examples and Examples

| | |
|---|---|
| MOPS: | morpholinopanesulfonic acid |
| LB medium: | Luria-Bertani medium |
| DMSO: | dimethylsulfoxide |
| PFU: | plaque forming unit |
| EDTA: | ethylenediaminetetraacetic acid |
| SDS: | sodium dodecyl sulfate |
| BRL: | Bethesda Research Laboratories Inc. |
| DMT: | dimethoxytrityl |
| lac: | lactose |
| Tris: | tris(hydroxymethyl)aminomethane |
| XAR-5: | X-ray film manufactured and sold by Eastman Kodak Company, U.S.A. |
| $1 \times$ SSC: | 0.15 M NaCl + 0.015 sodium citrate, pH7 |
| $2 \times$ SSC: | 0.30 M NaCl + 0.030 M sodium citrate, pH 7 |
| $3 \times$ SSC: | 0.45 M NaCl + 0.045 M sodium citrate, pH 7 |
| $5 \times$ SSC: | 0.75 M NaCl + 0.075 M sodium citrate, pH 7 |
| $6 \times$ SSC: | 0.9 M NaCl + 0.09 M sodium citrate, pH 7 |
| FDSS: | 50% deionized formamide + $5 \times$ Denhardt's + $5 \times$ SSPE + 0.1% SDS + 100 $\mu$g/ml denatured calf thymus DNA |
| SSPE: | 0.18 M NaCl + 10 mM $NaH_2PO_4$ + 1 mM EDTA, pH 7.4 |
| SM: | phage storage medium which contains 5.8g of NaCl 2 g of $MgSO_4 \cdot 7H_2O$, 50 ml of 1 M Tris $\cdot$ Cl (pH 7.5) and 5 ml of 2% gelatin per liter |
| NZ-broth: | medium which contains 10 g of NZ amine, 5 g of NaCl and 2 g of $MgSO_4 \cdot 7H_2O$ (NZ amine is a Type-A hydrolysate of casein manufactured and sold by Humko Sheffield Chemical Division of Kraft, Inc., U.S.A.) |
| IPTG: | isopropyl thiogalactoside |
| x-gal | : 5-dibromo-4-chloro-3-indolylgalactoside |
| TAE: | 0.04 M Tris-acetate (pH 8.0) - 0.002 M EDTA |
| $5 \times$ Denhardt's solution: | an aqueous solution containing Ficoll 1000 mg, polyvinyl-pyrrolidone 1000 mg and BSA 1000 mg per liter |
| bp: | base pair |

Referential Example 1

Step 1

(Preparation of TNF from rabbit serum)

Female rabbits, weighing 2.5 to 3 kg, are injected with 50 mg of formalin-killed *Propionibacterium acnes (Corvnebacterium parvum*; Wellcome Research Laboratories, England) through the ear vein. Eight days later, 100 $\mu$g of endotoxin (lipopolysaccharide from *Escherichia coli* 026:B6, produced by Difco Laboratories, U.S.A.) is injected again through the ear vein and 2 hours later whole blood is collected from the heart. To the collected blopod, heparin sodium is added in an amount of 100 units per 100 ml. The blood is then centrifuged while cooling at 5,000 rpm for 30 minutes to remove blood cells and insoluble solids. As a result, a plasma (2.4 liters) having a serum TNF cytotoxic activity of $3 \times 10^4$ units/ml is obtained from 40 rabbits.

Step 2

(Partial purification of TNF from rabbit serum)

To the plasma (2.4 liters) obtained in Step 1, added is 24 g of cellite. The resultant is stirred for one hour, and then subjected to filtration. The filtrate is mixed with 1.2 liters of 0.04 M Tris-HCl buffer (pH 7.8), and then applied to the column of DEAE-Sepharose CL-6B (manufactured and sold by Pharmacia Fine Chemicals, Inc. Sweden) sufficiently equilibrated with 0.04 M Tris-HCl buffer (pH 7.8) containing 0.1 M NaCl. The column is washed with 0.04 M Tris-HCl buffer, and the adsorbed TNF is eluted with 0.04 M Tris-HCl buffer (pH 7.2) containing 0.18 M NaCl. Fractions exhibiting cytotoxic activities against L cells are concentrated by ultrafiltration. The so obtained concentrate is applied to the column of Sephacryl S-200 (manufactured and sold by Pharmacia Fine Chemicals, Inc. Sweden) sufficiently equilibrated with 5 mM phosphate buffer and gel-filtered using the same buffer. The active fractions are concentrated by ultrafiltration, whereby a purified TNF having an activity of $3.5 \times 10^6$ units and a specific activity of $18 \times 10^6$ units/mg is obtained.

Step 3

(Anti-TNF antibody)

The rabbit serum TNF partially purified in Step 2 is mixed with complete Freund's adjuvant (1:1), and then injected subcutaneously at the back of a 12 week age BALB/c male mouse. The above operation is repeated 2 and 4 weeks after the initial injection. One week after the last injection, whole blood is collected. From the collected blood, a serum is obtained.

The so-obtained serum is added to the culture medium for evaluation of the cytotoxic activity of TNF against L cells in such an amount that it is diluted 500-fold in final concentration. The cytotoxic activity of the rabbit serum TNF against L cells is evaluated in the same manner as described before. It is found that the rabbit serum TNF exhibits no cytotoxicity against L cells. From the above result, it can be concluded that the mouse serum obtained in this step contains an antibody to the rabbit serum TNF (hereinafter referred to as "anti-TNF antibody").

Step 4

(Preparation of TNF-producing cells)

A female rabbit is injected intravenously with formalin-killed cells of *Propionibacterium acnes (Corynebacterium parvum;* Welcome Research Laboratories, England). Seven days later, the rabbit is subjected to tracheotomy, and the lung is washed with a physiological saline solution, whereby floating cells are obtained. The so obtained cells are washed with a physiological saline solution. Using as a culture medium RPMI 1640 (Flow laboratories Inc., U.S.A.) containing 10 v/v% fetal calf serum, the cells are incubated at 37°C in air containing 5% carbon dioxide. The cell culture is divided into two groups, and to one of them endotoxin derived from *Escherichia coli* (lipopolysaccharide from *Escherchia coli* 026:B6, produced by Difco Laboratories, U.S.A.) is added at a concentration of 10 $\mu$g/ml. The same amount of sterile water is added to the other. The supernatant of the cell culture to which endotoxin is added exhibits cytotoxic activity against L cells, and the activity reaches the maximum value within seven hours. Such activity is dissipated by the anti-TNF antibody, but is not dissipated by the normal mouse serum.

On the other hand, the supernatent of the cell culture to which no endotoxin is added exhibits no cytotoxicity against L cells.

Step 5

(Molecular weight of TNF)

To the cell culture prepared in Step 4 to which endotoxin is added radioactive L-[$^{35}$S] methionine (1300 Ci/mmol, produced by Amersham Industries plc, England) is further added (1 mCi/ml). In accordance with the method of Laemmli (see Laemmli, U.K. (1970), Nature (London), Vol 227, pp 680-685], the supernatant is analyzed by the SDS-polyacrylamide gel electrophoresis. The gel concentration is adjusted to 12.5 wt%. After the electrophoresis, the gel is treated with ENHANCE® (trademark of a product of New England Nuclear Inc., U.S.A.), and after drying, is exposed to X-ray film (Fuji RX, manufactured and sold by Fuji Photo Film Co., Ltd., Japan). In the supernatant of the cell culture in the presence of endotoxin, it is observed that a substance having a molecular weight of about 17500 is formed.

Further, the supernatant of each cell culture prepared in Step 4 is subjected to SDS-polyacrylamide gel electrophoresis in the same manner as described above. Thereafter, the gel is shaken in 2.5% NP 40® (a surface active agent sold by Calbiochem, U.S.A.) for one hour, and then in water for two hours. After shaking, each migration lane is separated by cutting, and cut into strips of 2 mm-width in a direction perpendicular to the direction of migration. Each strip is cultured with L cells, and evaluated for cytotoxic activity against L cells. In the lane on which the supernatant of the cell culture containing endotoxin is developed, cytotoxicity against L cells is observed at a position corresponding to the molecular weight of 17500. No cytotoxicity is observed at other positions.

Step 6

(Extraction of mRNA)

The cell culture as prepared in Step 4 is incubated for 2 hours after addition of endotoxin, followed by centrifugation to collect cells. Extraction of cytoplasmic RNA from the collected cells and extraction of mRNA from the cytoplasmic RNA are effected in accordance with the method of Chirgwin et al [see Chirgwin, J. M. et al, Biochemistry, Vol. 18, p. 5294 (1979)]. 4 ml of a 4 M guanidine thiocyanate solution is added to 3 × 10$^8$ cells, and the mixture is pulverized by means of a homogenizer (Model: AM-7, manufactured and sold by Nihon Seiki Seisakusho, Japan). The residues are removed by centrifugation, and 2.4 g of cesium chloride is dissolved therein. The mixture is carefully poured into a polyallomer tube in which 2.5 ml of 5.7 M cesium chloride and 0.1 M EDTA solution (pH 7.5) has been loaded in advance, and then subjected to ultracentrifugation at 30,000 rpm for 12 hours at 20°C using Beckman SW41 rotor (manufactured and sold by Beckman Instrument, U.S.A.). After removal of the supernatant, the pellet is dissolved in 1 ml of 10 mM Tris-HCl buffer (containing 5 mM EDTA and 1 w/v% SDS). The resulting solution is extracted with a 4:1 by volume mixture of chloroform and 1-butanol. To the aqueous phase, 0.05 volume of 2 M sodium acetate and 2.5 volumes of ethanol are added, and allowed to stand at -20°C for 2 hours or more, thereby to precipitate RNA. The precipitate is collected by centrifugation, dried, and then dissolved in 500 μl of sterile water. As a result, a cytoplasmic RNA solution is obtained.

The above-obtained RNA solution is heated at 68°C for 2 minutes, and thereafter, chilled quickly. 500 μl of 2-fold concentration 10 mM Tris-EDTA buffer (pH 7.4) (containing 1 mM EDTA, 0.1 w/v% SDS and 0.5 lithium chloride) is added to the solution, and the mixture is applied to a 200 mg oligo dT-cellulose (manufactured and sold by Bethesda Research Laboratories, Inc., U.S.A.) column, and washed with 10 ml of the same buffer (one-fold concentration) as described above. The material retained by the column is eluted with 2 ml of an elution buffer containing 10 mM Tris-HCl buffer pH 7.4, 1 mM EDTA and 0.1 w/v% SDS. To the eluate, is added 0.05 volume of sodium acetate and 2.5 volumes of ethanol, and the mixture is cooled at -20°C to precipitate. The precipitate is collected by centrifugation, and applied to the oligo dT-cellulose column, and the fractions adsorbed onto the oligo dT-cellulose are collected. 85 μg of mRNA is recovered as determined by the ultraviolet spectrum analysis.

Step 7

(Size fractionation of mRNA)

880 μg of mRNA prepared by the same method as described in Step 6 is dissolved in 250 μl of water, and the resulting solution is layered onto a 10 ml 5-25% linear sucrose density gradient. The surcrose density gradient is prepared by means of ISCO 570 gradienter (manufactured and sold by ISCO Inc., U.S.A.), using Tris buffer solutions (containing 25 mM Tris-HCl (pH 7.2), 2 mM EDTA and 1 w/v% SDS] respectively containing 5% sucrose and 25% sucrose.

Using Beckman SW41 rotor, ultracentrifugation is effected at 40000 rpm for 12 hours at 4°C, and fractions each of 400 μl are recovered by means of a fraction recovering apparatus (manufactured and sold by Beckman Instrument, U.S.A.), and then ethanol precipitated. The precipitated fractions are centrifuged, and dissolved in sterile water.

Step 8

(Experiment on translation of mRNA)

Translation of mRNA using ooctyes of Xenopus laevis (Hamamatsu biological teaching materials) is conducted according to the procedure described in the experimental reports (for example, Hiroshi Teraoka, Mikio Itsuki and Kentaro Tanaka, "Protein, Nucleic acid, Enzyme", Genetic Engineering, extra edition., 1981, p 602). Xenopus laevis is procured from Hamamatsu biological teaching materials. Fractionated mRNA obtained in Step 7 is dissolved in sterile water to have a concentration of 1 μg/μl, and the solution is injected into ooctyes in such a small amount as 50 nl per cell. Cells are then cultured for 24 houurs in a Barth's solution [containing 7.5 mM Tris-HCl (pH 7.6), 88 mM NaCl, 1 mM potassium chloride, 0.33 mM calcium nitrate, 0.41 mM calciuum chloride, 0.82 mM magnesium sulfate, 2.4 mM sodium bicarbonate, 18 U/ml penicillin G and 18 μg/ml streptomycin] which contains 1 mg/ml bovine serum albumin. Ooctes are crushed, in the culture liquid, by means of a glass bar. The culture liquid is then centrifuged, and the supernatant is evaluated for the cytotoxic activity against L cells. mRNA which will be translated to give a polypeptide having maximum activity sediments as 16 S in size. This activity is eliminated by the anti-TNF antibody obtained in Step 3, but is not eliminated by the normal mouse serum.

Step 9

(Preparation of transformants)

Using 5 μg of the fractionated mRNA obtained in Step 7, a double stranded DNA is prepared in accordance with procedure described in Literature (1), from page 96. As the reverse transcriptase, use is made of a product of Life Science, Inc., U.S.A. The double stranded DNA is size-fractionated on a 3.5% polyacrylamide gel, and 330 ng fraction of about 1000 to 2000 bp is obtained. In accordance with the procedure described in Literature (1), 7 ng of this fraction is extended with deoxyC residues using terminal deoxynucleotidyl transferase (manufactured and sold by Bethesda Research Laboratories, Inc., U.S.A.) and annealed with 56 ng of plasmid pBR322 which has been digested with PstI and extended with deoxyG residues. The so-annealed mixture is inserted into E. coli K-12 strain (HB101, ATCC 33694) to transform the strain. As a result, 12000 transformants are obtained.

Step 10

(Partial amino acid sequence of rabbit TNF)

Rabbit TNF partially purified in Step 2 (activity: $5 \times 10^7$ units) is subjected to SDS-polyacrylamide gel electrophoresis for purification as in Step 5. Part of the gel is dyed with Coomassie Brilliant Blue. A band at the position corresponding to the molecular weight of 17000 is cut out from the gel, and extracted with 1% ammonium bicarbonate. About 180 μg of TNF is recovered as protein.

150 μg of the recovered TNF is dissolved in 75 μg of 1% ammonium bicarbonate, followed by addition of 3 μg of TPCK trypsin (manufactured and sold by Worthington Biochemical, U.S.A.). The mixture is incubated at 37°C for 4 hours. The mixture is then fractionated by means a high-performance liquid chromatography column comprising Cosmosil 5C8 (manufactured and sold by Nakarai Chemical, Ltd.,

Japan) as the packing material, thereby to obtain fragments digested with trypsin.

The highly purified TNF and the trypsin-digested fragments thereof are then subjected to desalting by means of Sephadex G-25 column, and then freeze-dried. According to the method of R. M. Hewick et al (see J. Biol. Chem., Vol. 256, pp 7990-7997, 1981), the purified TNF and the trypsin-digested fragments are each subjected to Edman Degradation from the N-terminal. PTH-amino acid liberated in each step is analyzed by the customary method by means of a high-performance chromatography model SP 8100 (manufactured and sold by Spectra physics, U.S.A.) using Solpacks ODS (manufactured and sold by E. I. Du pont, U.S.A.); as the column. As a result, it is found that the TNF has the following N-terminal amino acid sequence:

Ser-Ala-Ser-Arg-Ala-Leu-Ser-Asp-Lys-Pro-Leu-Ala-

His-Val-Val-Ala-Asn-Pro-Gln-Val-Glu-Gly-Gln-Leu-Gln-

One of the trypsin-digested fragments has the following N-terminal amino acid sequence.
Glu Thr Pro Glu Glu Ala Glu Pro Met Ala

Step 11

(Synthesis of oligodeoxynucleotide probe)

Oligodeoxynucleotides complementary to the base sequence of the mRNA which is deduced from the amino acid sequence of rabbit TNF obtained in Step 10 is synthesized according to the improved phosphotriester method which has already been reported by the present inventor in H. Ito et al, "Nucleic Acid Res." *10*, 1755-1769 (1982). In preparing oligodeoxynucleotides, 128 oligodeoxynucleotides estimated from the amino acid sequence of rabbit TNF are classified into five groups, namely groups of 16, 16, 32, 32 and 32 and are synthesized as mixtures of oligodeoxynucleitides of the respective groups. The obtained oligodeoxynucleotides of the respective groups are deprotected according to the customary method and purified by column chromatography using Sephadex G-50 (manufactured and sold by Pharmacia Fine Chemicals, Inc., Sweden), electrophoresis on a 20% by weight polyacrylamide gel containing 7 M of urea and column chromatography using DE52 (manufactured and sold by Whatman Ltd., U.S.A.). The thus obtained oligodeoxynucleotides of the respective groups are dialyzed against 0.1 mM Tris-EDTA buffer solution.

Each of the purifed oligodeoxynucleotides of the respective groups is labelled using $T_4$ polynucleotide kinase (manufactured and sold by Bethesda Research Laboratories, Inc., U.S.A.) and $\gamma$-$^{32}$P-adrenosine triphosphate according to the customary method and then purified by column chromatography using DE52 (manufactured and sold by Whatman Ltd., U.S.A.). The radioactive material is incorporated into each of oligodeoxynucleotides of the respective groups in an amount of about $3 \times 10^8$ cpm/$\mu$g. The oligodeoxynucleotide probes each obtained in the form of a mixture of the respective groups are designated as shown in Table 1.

Part of the amino acid sequence of the rabbit TNF, the base sequence of the mRNA estimated from the amino acid sequence of the rabbit TNF and the base sequences of synthetic oligodeoxynucleotide probes of the respective groups are shown in Table 1.

TABLE 1

| Amino acid sequence | Carboxyl terminal . . . | Ala | Met | Pro | Glu | Ala | Glu | Glu | Amino . . . terminal |
|---|---|---|---|---|---|---|---|---|---|
| m RNA | 3′ . . . . . | XCG | GTA | XCC | YAG | XCG | YAG | YAG | . . . . . 5′ |
| Probe MH | 5′ | GC | CAT | MGG | MTC | GGC | MTC | MTC | 3′ |
| Probe MI | 5′ | GC | CAT | NGG | MTC | GGC | MTC | MTC | 3′ |
| Probe MJ | 5′ | GC | CAT | ZGG | MTC | AGC | MTC | MTC | 3′ |
| Probe K | 5′ | GC | CAT | ZGG | MTC | CGC | MTC | MTC | 3′ |
| Probe L | 5′ | GC | CAT | ZGG | MTC | TGC | MTC | MTC | 3′ |

Note: X represents a ribonucleic acid residue of A, C, G or U.
Y represents a ribonucleic acid residue of A or G.
M represents a deoxyribonucleic acid residue of T or C.
N represents a deoxyribonucleic acid residue of A or G.
Z represents a deoxyribonucleic acid residue of A, C, G or T.

Step 12

(Examination of oligonucleotide)

mRNA of the cells producing TNF which is obtained according to Step 6 is treated with a solution containing 1 M of glyoxal, 10 mM of $NaH_2PO_4$ and 50% by volume dimethyl sulfoxide at 50°C for 60 minutes and then subjected to fractionation using electrophoresis on a 1.1% by weight agarose gel. The fractionated mRNA is transferred on a filter of an electrophoresis type transfer blotting apparatus (manufactured and sold by Bio Rad, U.S.A.) according to the manual of the maker. Then the mRNA on the filter of the apparatus is treated with a 5 × Denhardt's solution containng a 5 × SSC solution and 150 μg/ml of denatured salmon spermatozoa DNA at 65°C for two hours and, then treated with a 5 × Denhardt.s solution containing 1 × 10⁷ cpm/ml of the labelled oligodeoxynucleotides and a 5 × SSC solution at 50°C for two hours. The above-obtained filter is washed with a 6 × SSC solution successively four times at room temperature, 40°C, 50°C and 60°C. An XAR-5 X-ray film (manufactured and sold by Eastman Kodak Company, U.S.A.) is exposed to the radiation from the filter. As a result, it is found that the oligodeoxynucleotides designated by Probe MJ are most strongly hybridized with the mRNA, showing that the oligodeoxynucleotide having a base sequence which is completely complimentary to the mRNA is contained in the oligodeoxynucleotides designated by Probe MJ.

Step 13

(Cloning of TNF gene of rabbit)

In accordance with the procedure described in Literature (2), page 162, the transformants obtained in Step 9 of are transferred onto a cellulose filter and the DNA of the transformants is hybridized with the labelled oligodeoxynucleotide (Probe MJ) selected in Step 12 under the same conditions as in Step 12 (colony hybridization). In the just above procedure, 49 colonies which are strongly hybridized with the labelled oligodeoxynucleotides (Probe MJ) are selected and further fixed onto another nitrocellulose filter. Then, using 49 colonies, further hybridization is carried out to select nine colonies which are more strongly hybridized with the labelled oligodeoxynucleotides (Probe MJ).

In accordance with the rapid plasmid separating procedure described in Literature (1), page 6, about 5 μg plasmid is obtained from each of the nine colonies. Each of the obtained plasmids is cleaved using restriction enzymes, *Pst*I, *Taq*I, *Rsa*I and *PVu*II (each manufactured and sold by Bethesda Research Laboratories, Inc., U.S.A.) according to the procedure described in the manual of the maker, followed by

15

electrophoresis effected on a 1% by weight agarose gel. Then, fragments obtained by cleavage by the respective restriction enzymes are compared with respect to length thereof.

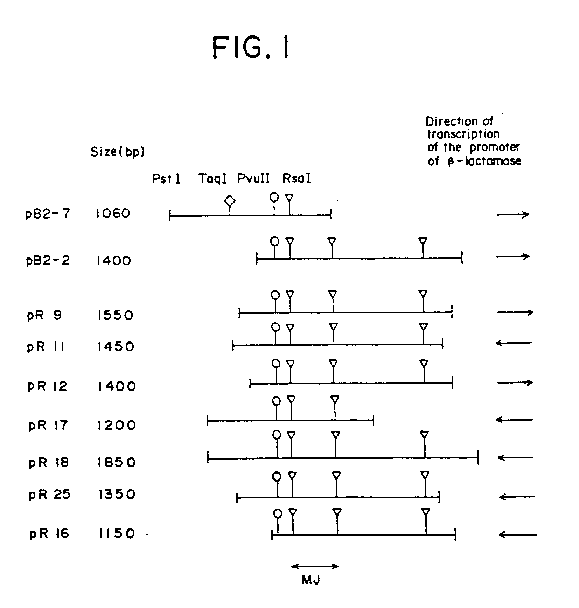

The results suggest that all the nine strains corresponding to the nine colonies have the base sequence of the fragment obtained by cleavage by *Pvu*II and *Rsa*I and consisting of about 50 bp and that most of the nine strains have the base sequence of the fragment obtained by cleavage by *Rsa*I and consisting of about 200 bp. In other words, the results suggest that the nine strains have partially common base sequences. The results of analysis by the restriction enzymes are shown in Fig. 1.

Seven strains containing plasmids designated in Table 2 below are separately cultivated in 2 ml of LB medium containing 10 μg/ml of tetracycline until the optical density of the solutions shows the values shown in Table 2 below, followed by centrifugation to obtain respective strains. Each of the obtained strains is separately added into 2 ml of physicological saline and disrupted by sonication. The obtained solutions are subjected to centrifugation and the cytotoxic activity against L cells of the obtained supernatants is determined. The results are shown in Table 2 below. As a blank test, the same procedures as mentioned above are repeated using a strain containing plasmid pBR322. The results are also shown in Table 2 below.

## TABLE 2

| Plasmid | Number of annealed base pairs | $OD_{600}$ | Cytotoxic activity against L cells (units/ml) |
|---------|------------------------------|------------|----------------------------------------------|
| pB 2—2 | 1400 | 1.369 | 35 |
| pB 2—3 | 800 | 1.605 | < 10 |
| pB 2—7 | 1060 | 1.364 | < 10 |
| pR 9 | 1550 | 1.618 | < 10 |
| pR 12 | 1400 | 1.458 | 15 |
| pR 18 | 1850 | 1.438 | < 10 |
| pR 25 | 1350 | 1.514 | < 10 |
| pBR322 | 0 | 1.677 | < 10 |

The cytotoxic activity against L cells is eliminated by anti-TNF antibody but is not eliminated by normal mouse serum. This shows that all of the above-mentioned nine colonies have plasmids which contain oligodeoxynucleotides coding for TNF.

Step 14

(Determination of base sequence of DNA coding for rabbit TNF)

*E. coli* strains containing plasmids pB2-7 and pR 18 are cultivated in one liter of M9 medium described in Literature (3), page 440 and containing 10 μg/ml of tetracycline. Then, in accordance with procedure described in Literature (3), page 90, each of the plasmids is isolated in an amount of about 150 μg.

The base sequence of the insert of each plasmid is determined according to the Maxam-Gilbert chemical procedure described in Maxam et a "Method in Enzymology", *55*, P 490 (1980), Academic Press. The thus determined base sequence is found to be in agreement with the partial amino acid sequences determined in Step 9. Thus, the whole sequence of TNF of rabbit is considered to be elucidated.

Step 15

In this step, construction of a plasmid is carried out using the recombinant plasmid pR12 to obtain direct expression of TNF in *E. coli* using *lac* as a promoter. The procedures are illustratively shown in Fig.

16

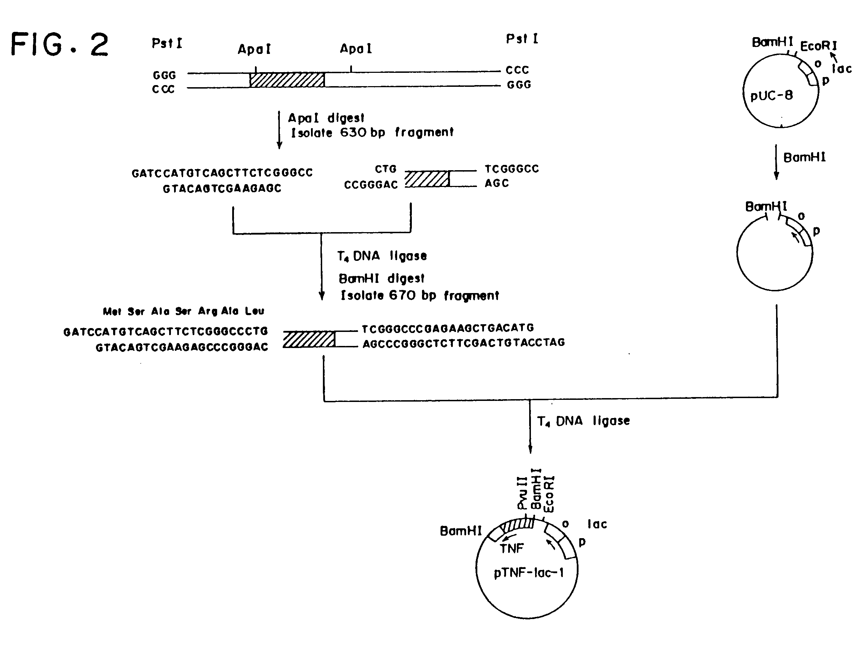

2. First 10 $\mu$g of plasmid pR12 is digested with 10 units of *Apa*I (manufactured and sold by Bethesda Research Laboratories, Inc., U.S.A.) at 37°C for two hours and electrophoresed on a 4% by weight polyacrylamide gel to isolate 610 bp fragments. About 1 $\mu$g of the fragment is isolated from the gel by electroelution. In the same manner as in Step 10, two oligodeoxynucleotides shown in Fig. 2 namely 5'-GATCCATGTCAGCTTCTCGGGCC-3' and 5'-CGAGAAGCTGACATG-3' are synthesized. Then, each 5' end of the oligodeoxynucleotides (about 100 pmole) is phosphorylated using $T_4$ polynucleotide kinase in accordance with the method described in Literature (3), page 122. After completion of the reaction, the reaction mixture is extracted with phenol and then with chloroform. Then the obained synthetic oligomers are mixed with 0.5 $\mu$g of the *Apa*I 630 bp fragment and ethanol precipitated. The fragment is ligated with the synthetic oligomers at 4°C overnight using 10 units of $T_4$ DNA ligase in accordance with the procedure described in Literature (1), page 37. After completion of the reaction, the reaction mixture is ethanol precipitated and digested with 20 units of *Bam*HI at 37°C for three hours, followed by electrophoresis effected on a 4% by weight polyacrylamide gel to recover 670 bp fragment by electroelution. One $\mu$g of commercially available plasmid pUC-8 (catalog No. 4916, manufactured and sold by P-L Biochemicals, Inc., U.S.A.) is digested with *Bam*HI and extracted with phenol and then with chloroform, followed by ethanol precipitation to obtain a vector. 0.5 $\mu$g of the obtained vector is ligated with the above-obtained fragment having *Bam*HI sites on its both ends and containing about 670 bp coding for TNF using $T_4$ DNA ligase. In accordance with the procedure described in Literature (4), page 20, *E. coli* JM101 is transformed using the above-obtained vector and cultivated on an agar medium containing 1 mM of IPTG and 0.004% (w/v) of X-gal to obtain about 200 white colonies. Plasmid DNA is prepared from 100 of these transformants and digested with *Bam*HI. As a result, it is found that 15 plasmids contain the intended *Bam*HI fragment (about 670 bp). In order to examine the direction of insertion, the above 15 plasmids are digested with *Eco*RI having only one recognition site on its pUC-8 and *Pvu*II having only one recognition site on its about 670 base pair fragment part and electrophoresed on a 6% by weight polyacrylamide gel. As a result, it is determined that 7 plasmids have the intended fragment consisting of about 140 bp and that the direction of transcription of the *lac* promoter on pUC-8 is in agreement with that of the oligodeoxynucleotides coding for TNF.

DNA sequence analysis shows that these seven plasmids have the same sequence and have the desired neucleotide sequence at the junctions between the *lac* promoter, synthetic DNA and cDNA.

Step 16

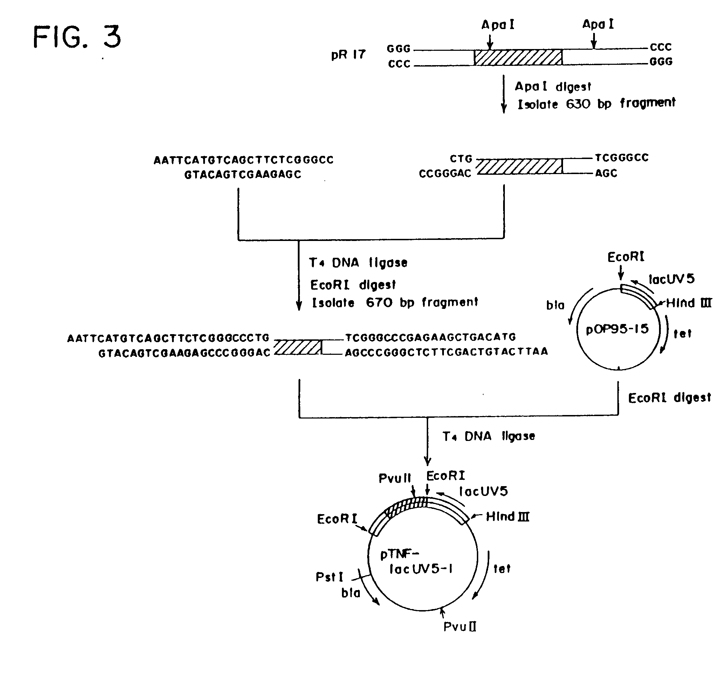

Construction of further plasmids is carried out using the recombinant plasmid pR17 in order to obtain direct expression of TNF in *E. coli* using *lac* UV5 as a promoter. The procedures are illustratively shown in Fig. 3. First, 10; $\mu$g of the plasmid pR17 is digested with 10 units of *Apa*I (manufactured and sold by Bethesda Research Laboratories, Inc., U.S.A.) at 37°C for two hours and electrophoresed on a 4% by weight polyacrylamide gel to isolate a fragment consisting of about 630 bp. About 1 $\mu$g of the fragment is isolated from the gel by electroelution. In the same manner as in Step 10, two oligodeoxynucleotides shown in Fig. 3, namely 5'-AATTCATGTCAGCTTCTCGGGCC-3' and 5'-CGAGAAGCTGACATG-3' are synthesized. Then, each 5' end of the two oligodeoxynucleotides (about 100 pmole) is phosphorylated using $T_4$ polynucleotide kinase in accordance with the method described in Literature (3), page 122. After completion of the reaction, the reaction mixture is extracted with phenol and then with chloroform. Then the synthetic oligomers are mixed with 0.5 $\mu$g of the previously obtained *Apa*I fragment (about 630 bp) prepared from the plasmid pR17 and ethanol precipitated. The fragment is ligated with the synthetic oligomers at 4°C overnight using 10 units of $T_4$ ligase in accordance with procedure described in Literature (1), page 37. After completion of the reaction, the reaction mixture is ethanol precipitated and digested with 20 units of *Eco*RI at 37°C for three hours, followed by electrophoresis effected on a 4% by weight polyacrylamide gel to recover a fragment (about 670 bp) by electroelution.

In accordance with the procedure described in F. Fuller, "Gene", *19*, pp 42-54 (1982), plasmid pOP95-15 is prepared.

One $\mu$g of pOP95-15 is digested with *Eco*RI and extracted with phenol and then with chloroform, followed by ethanol precipitation to obtain a vector. Using $T_4$ DNA ligase, 0.5 $\mu$g of the obtained vector is ligated with the fragment (about 670 bp) obtained by ligating the synthetic oligonucleotide with the oligonucleotide coding for TNF. In accordance with the procedure described in Literature (4), page 20, *E. coli* JM101 (ATCC 33876) is transformed using the above-obtained vector and cultivated on a medium containing 1 mM of IPTG and 0.004% (w/v) of X-gal to obtain about 150 white colonies. Plasmid DNA is prepared from 100 of these colonies and digested with *Eco*RI. As a result, it is found that 12 plasmids contain the intended *Eco*RI fragment (about 670 bp). In order to examine the direction of insertion, the above 12 plasmids are digested with *Pvu*II and *Pst*I and electrophoresed on a 1.5% by weight agarose gel. As a result, it is determined that four plasmids have the desired fragments (about 1280 bp and about 2600 bp) and that the direction of transcription of the *lac* UV5 promoter is in agreement with that of the oligodeoxynucleotides coding for TNF.

Base sequence analysis shows that these four plasmids have the same sequence and that the *lac* UV5 promoter, the synthetic oligodeoxynucleotide and cDNA are properly combined with each other. The obtained plasmids are designated pTNF-lacUV5-1.

Step 17

(Purification of TNF produced by *E. coli*)

*E. coli* strains containing plasmids obtained in Step 16 are cultivated on 50 ml of LB medium containing 100 μg/ml of ampicillin at 37°C overnight. Then the strains are transferred to 5 liter of LB medium containing 100 μg/ml of ampicillin and further cultivated at 37°C for three hours. Isopropyl-$\beta$-D-thiogalactopyranoside (manufactured and sold by Sigma Chemical Company, Inc., U.S.A.) is added to it to a final concentration of 1 mM. Further cultivation is carried out for six hours, followed by cooling. Then strains are collected by centrifugation. In the same manner as described in Step 13 the Strains are added into 5 liters of 0.04 M Tris-HCl buffer solution (ph 7.8) and disrupted by sonication to obtain a strain protein solution. The obtained solution has cytotoxic activity against L cells of $5 \times 10^7$ units/liter.

The obtained solution is purified in the same manner as Step 2 to obtain $1.2 \times 10^6$ units of TNF. The specific activity of the TNF is $6.8 \times 10^7$ units/mg.

Step 18

(Evaluation using transplanted Meth A sarcoma in mouse)

A sample (0.2 ml) of TNF obtained in Step 17 are subjected to evaluation by the in vivo assay method as described before.

Further, 20 Days after the injection of the sample, observations are made on the involution of tumors and recover rate is determined according to the following equation.

$$\text{Recovery rate} = \frac{\text{Number of mice which had been completely recovered from tumor}}{\text{Number of mice used for test}}$$

The results are shown in Table 3.

TABLE 3

| Injected amount of rabbit TNF produced by *E. coli* units/mouse | Number of mice used for test | Evaluation for activity of samples (after 1 day) | | | | Recovery rate (after 20 days) |
|---|---|---|---|---|---|---|
| | | - | + | + + | + + + | |
| $2 \times 10^5$ | 5 | 0 | 0 | 1 | 4 | 5/5 |
| Reference (physiological saline) | 5 | 5 | 0 | 0 | 0 | 0/5 |

18

Referential Example 2

Step 1

(Transformation of *E. coli* K12 Strain MC1061 with pR18, pB2-7 and pB2-2 Plasmids)

Colonies of *E. coli* K12 strain MC1061 are transformed with each of the pR18, pB2-7 and pB2-2 plasmids, which are obtained in Reference Example 3, according to the customary procedures. Specifically, colonies of *E. coli* K12 strain MC1061 are cultured in LB medium until the optical density of the culture broth becomes 0.3 at 550 nm. 50 ml of the grown *E. coli* culture is harvested, washed with a 25 ml mixture containing 10 mM MOPS (pH 7.0) and 10 mM RbCl, and resuspended in a 25 ml mixture containing 0.1 M MOPS (pH 6.5), 50 mM $CaCl_2$ and 10 mM RbCl. The resulting suspension is cooled on ice for 30 min, centrifuged and suspended in a mixture of 2 ml of the above-mentioned mixture containing 0.1 M MOPS (pH 6.5), 50 mM $CaCl_2$ and 10 mM RbCl and 30 $\mu$l of DMSO. To a 200 $\mu$l aliquot of the resulting suspension is separately added 10 $\mu$l of each of the plasmid DNA solutions. Each of the resulting mixtures is cooled on ice for 30 min, and then heat-shocked at 44°C for 60 seconds. Immediately thereafter, 5 ml of the LB medium pre-warmed at 37°C is added to each of the heated mixtures, followed by incubation at 37°C for one hour. The obtained culture broths are each subjected to centrifugation to form cell pellets. The supernatant is discarded, and LB medium is added and stirred to resuspend each of the cell pellets. Each of the resulting suspensions is inoculated to an LB agar plate containing 30 $\mu$g/ml tetracycline, followed by incubation at 37°C overnight. As a result, colonies of tetracycline-resistant transformants transformed, each, with pR18, pB2-7 and pB2-2 plasmids are obtained.

Step 2

(Preparation of pB2-7 and pR18 Plasmid DNAs)

Each of the transformants respectively transformed with pB2-7 and pR18 plasmids which are obtained in Step 1 is subjected to (1) growth of the transformant and amplification of the plasmid; (2) harvesting and lysis of the transformant, and (3) purification of the plasmid DNA, in accordance with the procedures as described at pages 88-96 of T. Maniatis, E. F. Fritsch and J. Sambrook, "Molecular Cloning", published by Cold Spring Harbor Laboratory, U.S.A. Illustratively stated, each of the transformants is inoculated into LB medium and incubated at 37°C with vigorous shaking. This step is repeated to attain growth of the transformant and amplification of the plasmid. The transformant culture is harvested by centrifugation at 4000 g for 10 min at 4°C. The supernatant is discarded. The resulting pellet is washed in 100 ml of ice-cold STE [0.1 M NaCl, 10 mM Tris•Cl (pH 7.8) and 1 mM EDTA], and subjected to lysis by boiling by the use of 20 mg/ml lysozyme in 10 mM Tris•Cl, pH 8.0. The viscous product is transferred to an ultracentrifuge tube, and centrifuged at 25,000 rpm for 30 min at 4°C to obtain a DNA solution. The volume of the DNA solution is measured. For every milliliter, exactly 1 g of solid cesium chloride is added and mixed gently until all of the salt is dissolved. 0.8 ml of a solution of ethidium bromide (10 mg/ml in $H_2O$) is added for every 10 ml of cesium chloride solution. The final density of the solution is 1.55 g/ml, and the concentration of ethidium bromide is approximately 600 $\mu$g/ml. The cesium chloride solution is transferred to a tube suitable for centrifugation, and the remainder of the tube is filled with light paraffin oil. Centrifugation is conducted at 45,000 rpm for 36 hours at 20°C to obtain two bands of DNA, the upper band thereof consisting of linear bacterial DNA and nicked circular plasmid DNA and the lower band thereof consisting of cloned circular plasmid DNA. The lower band of DNA is collected into a glass tube through a hypodermic needle inserted into the side of the tube. The ethidium bromide is removed, and the aqueous phase is dialyzed against TAE. The plasmid DNA solution is treated with RNase, and extracted with an equal volume of equilibrated phenol. The aqueous phase is layered on a column of Bio-Gel A-150 equilibrated in TAE (pH 8.0) and 0.1% SDS. The DNA in the column is washed, and a reservoir of TE with 0.1% SDS is applied to collect fractions. The fractions are precipitated with ethanol to obtain a pure plasmid DNA.

By conducting the above procedures, 250 $\mu$g of pure pB2-7 plasmid DNA and 134 $\mu$g of pure pR18 plasmid are obtained.

Step 3

(Nick Translation of Pure pB2-7 and pR18 Plasmid DNAs)

From the pure pB2-7 plasmid DNA obtained in Step 2, 40 $\mu$g is taken, digested with *Pst*I restriction enzyme and subjected to electrophoresis through 4% acrylamide gel. After electrophoresis, the DNA is stained and the desired band is cut out to isolate a *Pst*I insert.

Using 500 ng of the isolated *Pst*I insert, nick translation is carried out in the manner as described in Maniatis, T. et al, proc. Natl. Acad. Sci. U.S.A. *72*, 1184 (1975). For the nick translation, the Nick Translation Kit produced and sold by Bethesda Research Laboratories Inc., U.S.A. is employed, and 80 pmole of radioactive dCTP is applied in a 25-$\mu$l reaction system (at 400 Ci/mmole). To a mixture consisting of:

2.5 $\mu$l Solution A (dNTP's solution)
2.5 $\mu$l Solution B (500 ng of test DNA viz. *Pst*I insert)
5 $\mu$l hot dCTP (3200 Ci/mmole)
1.3 $\mu$l cold dCTP (65 pmole, 50 pmole/$\mu$l dCTP)
$\underline{11.2\ \mu l}$ Solution E ($H_2O$)
$\overline{22.5\ \mu l}$ (total)

is added 2.5 $\mu$l of Solution C (DNaseI, DNA Polymerase I), and reacted at 15°C for 60 min. Then, Solution D (stop buffer) is added to the resulting mixture to stop the reaction. Further, carrier tRNA is added, subjected to ethanol precipitation twice and dissolved in 500 $\mu$l of water. The specific activity per $\mu$g DNA is $9.3 \times 10^7$ cpm.

With respect to the pure pR18 plasmid DNA obtained in Step 2, also, the above-described procedures are carried out to effect the nick translation. The specific activity per $\mu$g DNA was $7 \times 10^7$ cpm.

Step 4

(Preparation of *Rsa*I Insert Fragment of pR18 Plasmid DNA)

80 $\mu$g of the pR18 plasmid DNA is digested with *Rsa*I restriction enzyme, and subjected to electrophoresis through 4% polyacrylamide gel. The following desired bands of inserts are cut out and purified by means of the BND column:

about 640 bp 3.77 $\mu$g (recovery 52%)
about 175 bp 1.77 $\mu$g (recovery 50%).

The above about 640 bp insert is designated as 3'-fragment of pR18 (meaning 3'-untranslated region of pR18), and the above about 175 bp insert is designated as pR18-cfr (meaning coding region of pR18).

Moreover the above procedures are repeated using *Pst*I and *Mst*II restriction enzymes instead of the *Rsa*I restriction enzyme to obtain the following band:

about 450 bp 3.65 $\mu$g (recovery 60%)

The above insert is designated as 5'-fragment of pR18.

Step 5

(Isolation of the Human Genomic TNF Gene)

The $^{32}$P-labelled plasmid pB2-7 insert obtained in Step 3 of Example 1 is used as a hybridization probe to screen $10^6$ plaques of bacteriophage Charon 4A/human genomic library prepared by insertion into the Charon 4A *Eco*RI ligation site [Blattner et al, "Science" *196*, 161 (1977)] of sized fragments from partially digested human DNA [Maniatis et al, "Cell" *15*, 687 (1978)]. The plaque hybridization method of Benton and Davis [Benton and Davis, "Science", *196*, 180 (1977)] is used. Since not all of the bacteriophage in the starting culture contain the necessary genetic material for preparing human TNF, a probe which has a base sequence complementary to the rabbit TNF gene is used. DNA of phage plaques having the desired genetic material incorporated the radioactive probe and are identified by their radioactivity. Nine hybridizing plaques are isolated from the library.

The procedures and conditions used are as follows.
1) Number of plaques:
~$1 \times 10^6$ plaques (~$4 \times 10^4$ plaques/$\phi$150 mm plate × 25)
2) Transfer to nitrocellulose filters:
[see Benton and Davis, Science, *196*, 186 (1977)]

20

3) Hybridization:

Addition of 1.25 × 10⁵ cpm/ml of pB2-7 insert probe prepared in Step 3 of Example 1, 42°C, 19.5 hr

4) Washing:

$$2 \times SSC - 0.1\% \text{ SDS at room temp.}$$
$$\text{Immersion 10 min.} \times 4$$
$$\downarrow$$
$$1 \times SSC - 0.1\% \text{ SDS at 50°C}$$
$$\text{Immersion 30 min.} \times 2$$

5) Exposure:

XAR-5 (Eastman Kodak Company, U.S.A.) -80°C, 2 intensifying screens, 39 hr

In the above screening, 12 candidate strains are obtained. In the same manner as mentioned above, second screening is carried out to obtain nine strains containing the intended fragment. Using these strains, third screening is carried out in the same manner as mentiond above to obtain nine strains containing the intended fragment. Using the obtained strains, fourth screening is carried out to confirm that the nine strains contain the intended fragment. The obtained nine bacteriophages containing the intended fragment are designated HG-1~HG-9, respectively.

Step 6

(Isolation of Rabbit Genomic TNF Gene)

Substantially the same procedure as described in Step 5 of Example 1 are repeated except that 10⁶ plaques of bacteriophage Charon 4 A/rabbit genomic library which is prepared using digested rabbit DNA [Maniatis et al, Cell, *15*, 687 (1978)] instead of digested human DNA. 6.7 × 10⁵ plaques of bacteriophage Charon 4A/rabbit genomic library are used instead of 10⁶ plaques of the bacteriophage Charon 4A/human genomic library. Thus, there is obtained two bacteriophage strains (RG-1 and RG-2) containing the rabbit genomic TNF gene.

Step 7

(Southern blotting analysis of human clones)

Using the bacteriophages HG-3, HG-6 and HG-7 obtained in Step 5 of Example 1, DNA of each bacteriophage is obtained according to the following procedures.

6 × 10¹⁰ cells of *E. coli* LE392 (host cell) are suspended in 18 ml of SM and 3 × 10⁹ PFU of bacteriophage HG-3 is added, thus allowing the *E. coli* to be infected at 37°C for 20 minutes. Then, the obtained mixture is added in 3 liters of NZ-broth and subjected to shaking culture at 37°C for 23 hours. 60 ml of $CHCl_3$ is added to the mixture and further subjected to shaking culture for 30 minutes. NaCl is added to the mixture to a final concentration of 1 M, the mixture is allowed to stand for 15 minutes, followed by centrifugation to obtain supernatant. Then, polyethylene glycol (molecular weight: about 6000) is added to the mixture so that the concentration of polyethylene glycol becomes 10% (w/v), and allowed to stand for 22 hours at 4°C. Bacteriophages are collected by centrifugation. The obtained bacteriophages are suspended in 28 ml of SM and an equal volume of $CHCl_3$ is added. After stirring by means of Vortex for 30 seconds, the mixture is subjected to centrifugation to obtain aqueous phase. SM is added to the aqueous phase so that the total amount becomes 30 ml. 26.4 g of CsCl is added to the obtained mixture and dissolved gently, followed by ultracentrifugation (45000 rpm, 20 hours) to obtain bacteriophages in the form of a band. The obtained mixture containing bacteriophages is dialyzed against 10 mM NaCl - 50 mM Tris (pH 8) - 10 mM $MgCl_2$. Then EDTA, Proteinase K and SDS are added to the mixture so that the concentrations of them are 20 mM, 50 $\mu$g/ml and 0.5% (w/v), respectively. Then the mixture is treated at 65°C for one hour and extracted with phenol, a mixture of phenol and $CHCl_3$ (1:1 by volume) and then with $CHCl_3$. The obtained aqueous phase is dialyzed against 10 mM Tris (pH 8) - 1 mM EDTA. The ultraviolet absorption measurement of the obtained aqueous phase shows that pure DNA of the bacteriophage HG-3 is obtained.

Substantially the same procedures as described with respect to the preparation of DNA of the bacteriophage HG-3 are repeated to obtain DNAs of bacteriophages HG-6 and HG-7.

Thus, there are obtained 2920 $\mu$g of HG-3, 1100 $\mu$g of HG-6 and 819 $\mu$g of HG-7.

In accordance with the Southern method [E. M. Southern, J. Mol. Biol., *98*, 503 (1975)], Southern blotting analysis of the obtained DNAs is performed. The procedures and conditions are as follows.

1) DNA:

HG-3 825 ng each

HG-6 935 ng each

HG-7 685 ng each

2) Digestion with various restriction enzymes:

10 units *Bam*HI, 10 units *Eco*RI,

10 units *Bam*HI + 10 units *Eco*RI

10 units *Hind*III,

10 units *Hind*III + 10 units *Eco*RI

10 units *Pvu*II

37°C, 3 hr

3) Electrophoresis:

0.8% Agarose gel

TAE

28 V, 15.5 hr

4) Transfer to nitrocellulose filters:

[see E. M. Southern, J. Mol. Biol., *98*, 503 (1975)]

5) Pre-hybridization:

30 ml FDSS

42°C, 6hr

6) Hybridization

5'-fragment (1 × $10^5$ cpm/ml) of pR18 (prepared in Step 4 of Example 1) 42°C, 14 hr

7) Washing:

2 × SSC − 0.1% SDS at room temp.
Immersion 10 min. × 4
↓
1 × SSC − 0.1% SDS at 50°C
Immersion 30 min × 2

8) Exposure

XAR-5 (Eastman Kodak Company, U.S.A.)

-80°C, 2 intensifying screens, 14 hr

The results of hybridization are shown in Table 4.

TABLE 4

| Enzyme | Clone (bacteriophage) | Hybridizing fragment size with Probe (pR18) | |
|---|---|---|---|
| | | 5' end | 3' end |
| *Bam*HI | HG-3<br>-6<br>-7 | 6.7 kb<br>11.2 kb<br>9.2 kb | ←<br>←<br>← |
| *Bam*HI + *Eco*RI | HG-3<br>-6<br>-7 | 2.9 kb<br>"<br>" | ←<br>←<br>← |
| *Eco*RI | HG-3<br>-6<br>-7 | "<br>"<br>" | ←<br>←<br>← |
| *Hind*III + *Eco*RI | HG-3<br>-6<br>-7 | "<br>"<br>" | ←<br>←<br>← |
| *Hind*III | HG-3<br>-6<br>-7 | 9.7 kb<br>4.1 kb<br>9.7 kb | ←<br>←<br>← |
| *Pvu*II | HG-3<br>-6<br>-7 | 2.2 kb<br>1,9 kb<br>2.2 kb | 0.9 kb<br>0.9 kb<br>0.9 kb |
| NOTE: The symbol "←" means same fragment hybridizes. | | | |

Step 8

(Southern blotting analysis of rabbit clones)

Substantially the same procedures as in Step 7 of Example 1 are repeated except that each of the bacteriophages RG-1 and RG-2 is used instead of each of the bacteriophages HG-3, HG-6 and HG-7. Thus, there is performed Southern blotting analysis. As a result, it is found that pR18 5'-fragment is hybridized with a single band fragment of fragments which are obtained by cleavage of RG-1 and RG-2 with each of *Bam*HI, *Eco*RI, *Bgl*II, *Hind*III and *Bam*HI + *Eco*RI.

Step 9

(Construction of bacterial clones containing human genomic TNF gene)

The method of Landy et al [Biochemistry, Vol. 13, 2134 (1974)] is used to obtain DNA of HG-3 as obtained in the above Step 5. 33 $\mu$g of the resulting HG-3 as obtained in the above Step 5. 33 $\mu$g of the resulting HG-3 DNA is digested with 80 units of *Eco*RI at 37°C for 3 hours. The digest is electrophoresed on 1% low melting agarose gel (conditions: 1 × TAE, 20 V, 14.5 hr). The 2.9 kb band is isolated from the agarose gel as described by T. Maniatis [Molecular Cloning, Cold Spring Harbor Laboratory, p 377 (1982)]. Specifically, the cut-out gel of the 2.9 kb band portion is heated at 65° for 15 min. The *Eco*RI-cleaved HG-3 fragment having a length of 2.9 kb (hereinafter often referred to as "HG-3/*Eco*RI 2.9 kb fragment") is recovered from the melted gel by extracting 3 times with phenol and then 3 times with ethanol, followed by precipitation with ethanol containing ammonium acetate. Thus, there is obtained 637 ng (yield: about 30%) of HG-3/*Eco*RI 2.9 kb fragment.

255 ng of the above-obtained fragment is ligated to 56.5 ng of *Eco*RI-cleaved pUC 13 [J. Messing, Methods in Enzymology, Vol. 101, 20 (1983)] using 2.5 units of T$_4$ ligase at 4°C for 20 hours.

*E. coli* K12 strain JM83 is transformed using the above-obtained ligation product. Specifically, *E. coli* K12 strain JM83 is cultured in LB medium until the optical density of the culture broth becomes 0.3 at 550 nm. 50ml of the grown *E. coli* K12 strain JM83 culture is collected, washed with a 25 ml of 10 mM MOPS (pH 7.0)-10 mM RbCl, and resuspended into a 25 ml of 0.1 M MOPS (pH 6.5)-50 mM CaCl$_2$-10 mM RbCl and 30 $\mu$l of DMSO. To 203 $\mu$l of the suspension is added 10 $\mu$l of an aqueous ligation product solution containing 10 ng of the ligation product. The mixture is cooled on ice for 30 min and then heated at 42°C for 60 seconds. Immediately thereafter, 5 ml of LB broth prewarmed at 37°C is added to the heated mixture, followed by incubation at 37°C for one hour. The obtained culture broth is subjected to centrifugation and the supernatant is removed. An LB medium is added to the resulting cell pellet and then inoculated on an LB plate containing 30 $\mu$g/ml ampicillin and 40 $\mu$g/ml X-gal. Colonies containing *E. coli* K12 strain JM83 which have been transformed with the plasmids having the insert are white, while those containing *E. coli* K12 strain JM83 which have been transformed with plasmid only are blue. The obtained white colonies are inoculated again on LB plate containing 30 $\mu$g/ml ampicillin and 40 $\mu$g/ml X-gal for the purpose of confirmation.

From the above-obtained white colonies ten colonies (bacterial clones) are selected and screened by using mini-prep technique of Holmes and Quigley [Anal. Biochem., Vol. 114, 193 (1981)].

Specifically, each colony is cultured overnight on medium containing 30 $\mu$g/ml ampicillin. The grown cells are collected and suspended in 2 mg/ml lysozyme-5.0 mM glucose-10 mM EDTA-25 mM Tris HCl (pH 8.0). The suspension is allowed to tand at room temperature for 5 minutes, followed by addition of 200 $\mu$l of 0.2 N NaOH-1% SDS. After slowly stirring, the suspension is allowed to stand at room temperature for 2 min. Thereafter, 150 $\mu$l of 3 M sodium acetate (pH 5.2) is added, allowed to stand at -20°C for 10 min, followed by centrifugation for 15 min to recover the resulting supernatant. To the supernatant is added 900 $\mu$l of cold ethanol, followed by centrifugation for 5 min to obtain the resulting precipitate. The obtained precipitate is washed with 70% ethanol and dried to get a plasmid DNA. In the above-mentioned method, ten plasmid DNAs are obtained.

Each plasmid DNA is dissolved in 10 mM Tris-0.1 mM EDTA (pH 8.0), digested with *Eco*RI and subjected to electrophoresis for restriction analysis. The conditions for digestion and electrophoresis are as follows.

*Digestion:* plasmid DNA solution, one-fifth of the amount as prepared above; *Eco*RI, 3 units; 37°C; 1.5 hr

*Electrophoresis:* 1% agarose gel; 1 × TAE; 120 V; 2 hr

The above restriction analysis shows that eight of ten clones are postive. That is, the eight clones have 2.9 kb fragment. From the eight positive clones one clone is selected and designated as *E. coli* K12 strain JM 83 (pHGE) (ATCC 39565).

Substantially the same procedures as in the above Step 2 are repeated to prepare 1.89 mg of pHGE DNA, except that *E. coli* K12 strain JM63 (pHGE) is used instead of *E. coli* harboring pB2-7 and pR17.

## Step 10

(Subcloning of *Eco*RI-cleaved RG-1)

30 $\mu$g of RG-1 as prepared in the above Step 6 is digested with *Eco*RI. From the resulting fragment mixture the fragment having a length of about 3.5 kb is recovered in substantially the same manner as in the above step 9, except that the above prepared fragment mixture and 0,8% low melting agarose gel are used. There is obtained 1.0 $\mu$g of *Eco*RI-cleaved RG-1 fragment (about 3.5 kb). The above-obtained *Eco*RI-cleaved RG-1 fragment (3.5 kb) is ligated to *Eco*RI-digested pUC13 in substantially the same manner as in the above step 9, except that the above-obtained *Eco*RI-cleaved fragment (3.5 kb) is used instead of *Eco*RI-cleaved HG-3 fragment (2.9 kb).

The transformation of *E. coli* K12 strain JM83, screening of bacterial clones, digestion of clones and electrophoresis are effected in substantially the same manner as in the above Step 9, except that the above-obtained ligation product is used. The obtained clone is designated as *E. coli* K12 strain JM83 (pRGE) (ATCC 39655).

Substantially the same procedures as in the above Step 2 are repeated to prepare 1.70 mg of - RGE DNA, except that *E. coli* K12 strain JM83 (pRGE) is used instead of pB2-7 and pR-18.

Step 11

(Restriction enzyme analysis of pHGE plasmid DNA)

The restriction enzyme analyzis of pHGE DNA as obtained in the above Step 9 is effected according to the method as described in Maniatis [Molecular Cloning, Cold Spring Harbor Laboratory, 98 (1982)].
The procedures and conditions used are as follows.
1) Digestion of pHGE DNA with *Eco*RI:
    18.6 $\mu$g pHGE DNA
    64 units *Eco*RI
    37°C, 2 hr
2) Ethanol precipitation: precipitate
3) Addition of distilled water to precipitate:
    Preparation of 1 $\mu$g/$\mu$l *Eco*RI-cleaved pHGE soln.
4) Digestion with various restriction enzymes:
    1 $\mu$g pHGE/*Eco*RI
    Restriction enzyme: 5 units *Pvu*II, 5 units *Pvu*II + 10 units *Rsa*I, 10 units *Rsa*I, 4 units *Mst*II, 3 units *Ava*I, 9 uinits *Pst*I
    37°C, hr
5) Electrophoresis:
    2% Agarose gel, 1 × TAE,
    28V, 14.5 hr
6) Transfer to nitrocellulose filter:
    [see E.M. Southern, J. Mol. Biol., *98*,503 (1975)]
7) First pre-hybridization:
    30 ml FDSS
    42°C, 6 hr
8) First hybridization:
    5'-fragment (5 × $10^4$ cpm/ml) of pR18
    (prepared in the above Step 4)
    42°C, 14 hr
9) Washing:

    2 × SSC − 0.1% SDS at room temp.
    Immersion 10 min. × 4
    ↓
    1 × SSC − 0.1% SDC at 50°C
    Immersion 30 min × 2

10) Exposure:
    XAR-5 (Eastman Kodak Company, U.S.A.), -80°C, 2 intensifying screens, 17.5 hrs
11) Washing out:

    0.5 NaOH − 1.5 M NaCl (Immersion: 1 min.)
    0.5 M Tris − 1.5 M NaCl (Immersion: 1 min.)
    ↓
    3 × SSC (Immersion: 1 min.)

12) Exposure:
    Effected in the same manner as in the above 10), except that exposure time is 19 hrs.
13) Second pre-hybridization:
    In the same manner as in the above 7)
14) Second hybridization:
    pB2-7 insert (prepared in the above Step 3),
    42°C, 16.5 hrs
15) Washing:
In the same manner as in the above 9)

16) Exposure:

In the same manner as in the above 10), except that exposure time is 10.5 hrs.

17) Washing out:

In the same manner as in the above 11)

18) Exposure:

In the same manner as in the above 10), except that exposure time is 20 hrs.

19) Third pre-hybridization:

In the same manner as in the above 7).

20) Third hybridization:

3' - fragment ($4.5 \times 10^5$ cpm/ml) of pR18 (prepared in the above Step 4), 42°C, 15 hr.

21) Washing:

In the same manner as in the above 9).

22) Exposure:

In the same manner as in the above 10).

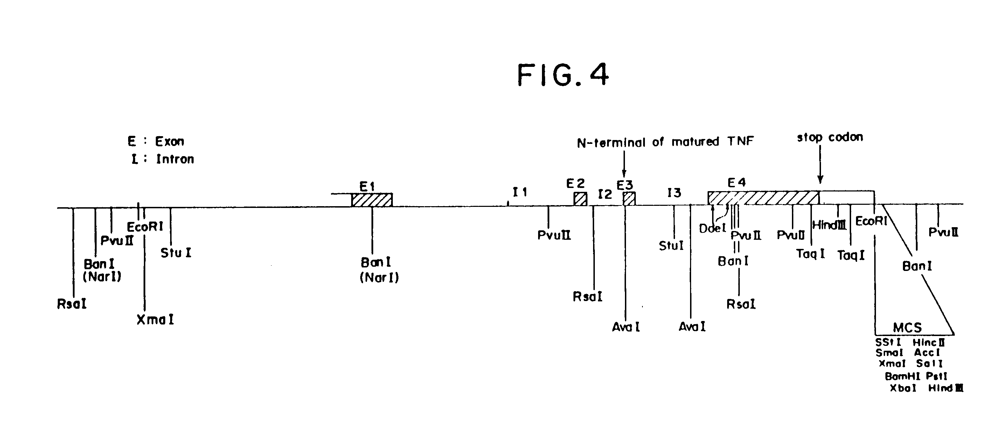

The results of the restriction enzyme analysis are shown in Fig. 4.

Step 12

(Restriction enzyme analysis of pRGE plasmid DNA)

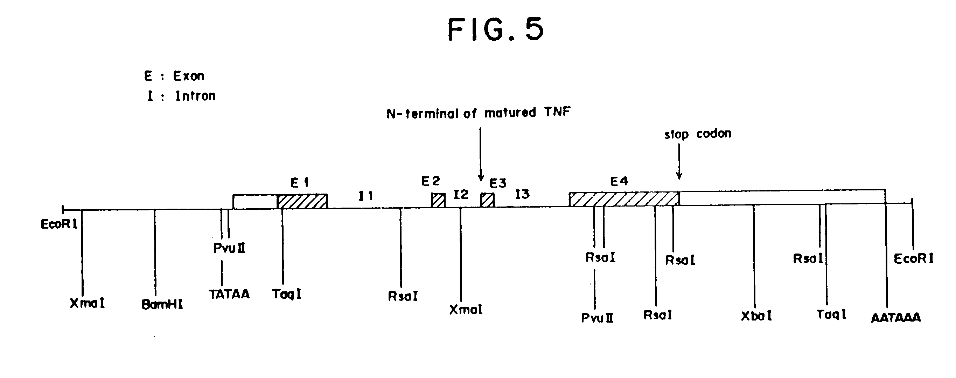

In substantially the same manner as in the above Step 11, the restriction enzyme analysis of pRGE plasmid DNA prepared in the above step 10 is effected except that pRGE plasmid DNA is used instead of pHGE plasmid DNA. The restriction map of pRGE DNA insert obtained is shown in Fig. 5.

Step 13

(Determination of base sequences of rabbit TNF gene and human TNF gene)

Substantially the same procedures as in the above Step 2 are repeated, except that *E. coli* K12 strain JM83 (pHGE) obtained in the above Step 9 and *E. coli* K12 strain JM83 (pRGE) obtained in the above Step 10 are used instead of *E. coli* K12 strain MC1061 having pB2-7 and *E. coli* K12 strain MC1061 having pR18. Thus, 150 µg of each of pRGE plasmid DNA and pHGE plasmid DNA is obtained.

The base sequences of pRGE and pHGE are determined according to the Maxam-Gilbert method (Maxam et al, Methods in Enzymology, Vol. 55, 490 (1980) published by Academic Press].

The base sequence of pR-18 determined in Referential Example 1 is compared with that of pRGE as determined above to elucidate the structure, including exon and intron, of rabbit TNF gene. The structure of pRGE DNA insert is shown in Fig. 5. Subsequently, the base sequence of pRGE is compared with that of pHGE to investigate the homology and consensus sequence around the boundary between intron and exon. Thus, the structure, including exon and intron, of human TNF gene is elucidated. The structure of human TNF gene is shown in Fig. 4.

The above-obtained base sequence coding for rabbit TNF and humsn TNF will be shown below. In the base sequences, the upper row shows the base sequence coding for rabbit TNF (R) and the lower row the base sequence coding for human TNF (H).

```
R   TCA GCT TCT CGG GCC CTG AGT GAC AAG CCT CTA GCC CAC GTA GTA
H   TCA TCT TCT CGA ACC CCG AGT GAC AAG CCT GTA GCC CAT GTT GTA

R   GCA AAC CCG CAA GTG GAG GGC CAG CTC CAG TGG CTG AGC CAG CGT
H   GCA AAC CCT CAA GCT GAG GGG CAG CTC CAG TGG CTG AAC CGC CGG

R   GCG AAC GCC CTG CTG CGC AAC GGC ATG AAG CTC ACG GAC AAC CAG
H   GCC AAT GCC CTC CTG GCC AAT GGC GTG GAG CTG AGA GAT AAC CAG

R   CTG GTG GTG CCG GCC GAC GGG CTG TAC CTC ATC TAC TCC CAG GTT
H   CTG GTG GTG CCA TCA GAG GGC CTG TAC CTC ATC TAC TCC CAG GTC

R   CTC TTC AGC GGT CAA GGC TGC CGC TCC ... TAC GTG CTC CTC ACT
H   CTC TTC AAG GGC CAA GGC TGC CCC TCC ACC CAT GTG CTC CTC ACC

R   CAC ACT GTC AGC CGC TTC GCC GTC TCC TAC CCG AAC AAG GTC AAC
H   CAC ACC ATC AGC CGC ATC GCC GTC TCC TAC CAG ACC AAG GTC AAC

R   CTC CTC TCT GCC ATC AAG AGC CCC TGC CAC CGG GAG ACC CCC GAG
H   CTC CTC TCT GCC ATC AAG AGC CCC TGC CAG AGG GAG ACC CCA GAG

R   GAG GCT GAG CCC ATG GCC TGG TAC GAG CCC ATC TAC CTG GGC GGC
H   GGG GCT GAG GCC AAG CCC TGG TAT GAG CCC ATC TAT CTG GGA GGG

R   GTC TTC CAG TTG GAG AAG GGT GAC CGG CTC AGC ACC GAG GTC AAC
H   GTC TTC CAG CTG GAG AAG GGT GAC CGA CTC AGC GCT GAG ATC AAT

R   CAG CCT GAG TAC CTG GAC CTT GCC GAG TCC GGG CAG GTC TAC TTT
H   CGG CCC GAC TAT CTC GAC TTT GCC GAG TCT GGG CAG GTC TAC TTT

R   GGG ATC ATT GCC CTG
H   GGG ATC ATT GCC CTG
```

Note: the symbol "..." means that this portion in the base sequence of the DNA coding for rabbit TNF is null and, therefore, two codons adjacent to this symbol at its both sides are directly connected.

Step 14

(Synthesis of oligodeoxynucleotides)

To a stainless steel 500 μl reaction vessel with stainless steel filters at each end is added 20 mg of a polystyrene resin to which a nucleoside (2.0 μM) is connected via a succinate linkage. The resin is treated with zinc bromide (1 M) in dichloromethane isopropanol (85:15) to remove the dimethoxytrityl (DMT) protecting group, washed with dimethylformamide, pyridine, and acetonitrile, and dried with a stream of nitrogen. To the dried resin is added a solution of DMT-nucleotide (20 μM) and mesitylenesulfonyl-nitrotriazole (60 μM) in 200 μl pyridine. The coupling reaction is allowed to proceed at 45°C for 20 minutes. This cycle of deprotection and coupling is repeated for successive nucleotides until the desired oligodeoxynucleotide is assembled on the resin. The resin is then treated to remove the oligodeoxynucleotide therefrom and purified as described by Ito, Ike, Ikuta, and Itakura (Nuc. Ac. Res. *10*:1755 (1982)).

Thus, the following oligodeoxynucleotides are obtained:

1) 5'-AATTCATGTCATCTTCTCGAACCCCGAGTGACAA-3'
2) 3'-GTACAGTAGAAGAGCTTGGGGCTCACTGTTCGG-5'
3) 5'-GCCTGTAGCCCATGTTGTAGCAAACCCTCAAGC-3'
4) 3'-ACATCGGGTACAACATCGTTTGGGAGTTCGACT-5'

Step 15

(Construction of M13mp9-HGE containing the human minigene for TNF)

Plasmid pHGE (10 μg) is digested with *Eco*RI(20 units). After electrophoresis on a 1% low-melting agarose gel, the 2.9 kb fragment is eluted. This fragment is inserted into *Eco*RI fragment from the replicative form of M13mp9 phage. The M13mp9 phage is selected because it is especially suited for receiving sections of DNA. The product transfects to *E. coli* JM103 (BRL (Bethesda Research Laboratories, Inc., U.S.A.) User Manual/M13mp7 Cloning/'Dideoxy' sequencing, 1980]. The product is designated M13mp9-HGE.

Step 16

(Deletion of Intron 3, using M13mp9-HGE single strand DNA and Deleter E3-4)

The single strand DNA of M13mp9-HGE is prepared by the method of BRL User Manual/M13mp7 cloning/'Dideoxy' sequencing, 1980.
Oligodeoxynucleotide 4)
        3'-ACA TCGGGTACAACATCGTTTGGGAGTTCGACT-5'
prepared in Step 14 is used as a deleter for the intron 3. The deleter for the intron 3 is designated "E3-4".
        The deleter E3-4 has a base sequence which is complementary to the base sequence of the bases before (Exon 3) and after (Exon 4) the intron 3 which is to be deleted. Deletion of the intron 3 is effected, in accordance with the teaching of Wallace et al, Science 209:1396 (1980), as follows.
        E3-4 (164 ng, 15 pmole) is phosphorylated using T4 kinase (10 units) and ATP (3mM) and added to the template M13mp9-HGE (1.65 μg, 0.5 pmole). The reaction mixture is heated at 65°C for 10 minutes, cooled to room temperature for 5 minutes, and finally cooled in ice water. To dATP, dCTP, dGTP, dTTP and ATP (0.4 mM), is added Klenow fragment (5 units), T4 ligase (10 units) in Hin buffer [Wallace et al, Nuc. Ac. Res. *9*; 3647 (1981)], 10 mM Tris HCl (pH 7.2), 2 mM MgC$_2$ and 1mM β-mercaptoethanol. The reaction mixture (final volume 50 μl) is incubated for 30 minutes at 4°C and then for 30 minutes at room temperature. The DNA from the oligonucleotide-primed reaction is used to transfect *E. coli* JM103 in accordance with the procedure of BRL User Manual/M13mp7 cloning/'Dideoxy' sequencing, 1980. Plaques obtained in this way are picked to YT plates [J. H. Miller, p. 433, Experiments in Molecular Genetics, Cold Spring Harbor Laboratory (1972)]. The colonies obtained are hybridized at 55°C for 2 hours with $^{32}$P-labelled E3-4. For this step, the deleter is used as a probe to identify sequences of DNA having the corresponding complementary base sequence after the intron has been deleted. Phage are isolated from those colonies which hybridize with the deleter.
        The resulting phage are plated and plaques are picked to YT plates. The clones are allowed to hybridize at 55°C for 2 hours with $^{32}$P-labelled E3-4. Positive clones are obtained and the phage DNA is sequenced to select those phage in which intron 3 is completely deleted. One such phage is designated mp9-HGEΔ 3-1.

Step 17

(Construction of pHTNF-lacUV5-2)

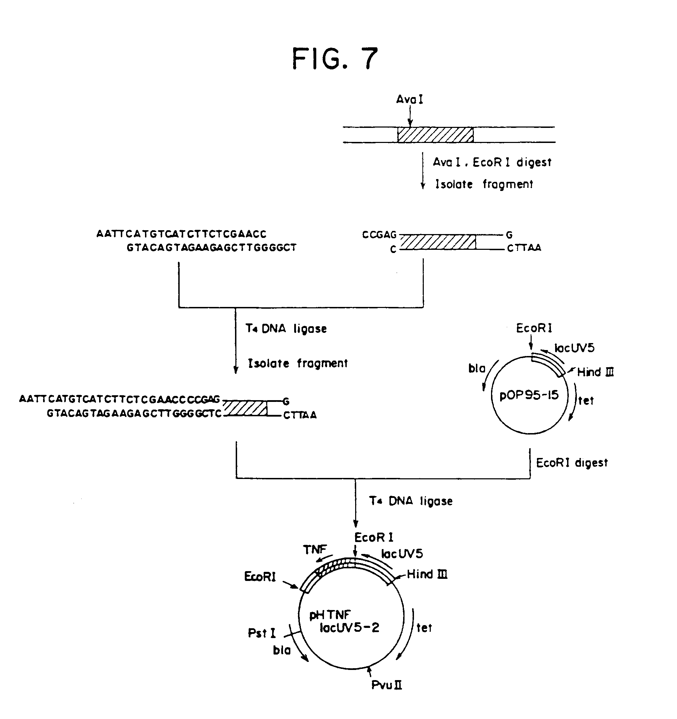

The replicative form of mp9-HGEΔ3-1 is digested with *Eco*RI. The *Eco*RI fragment is isolated and cloned to *Eco*RI-cleaved pBR327 to yield the plasmid pHGEΔA3-1.
        Construction of further plasmid is carried out using plasmid pHGEΔ3-1 in order to obtain such plasmid Δ3-1 as will directly express. TNF in *E. coli* using *lac* UV5 as a promoter. The procedures are illustratively shown in Fig. 7. First, 10 μg of plasmid pHGEΔ3-1 is digested with 10 units of *Ava*I and *Eco*RI (manufactured and sold by Bethesda Research Laboratories, Inc., U.S.A.) at 37°C for two hours and electrophoresed on a 4% by weight polyacrylamide gel to isolate fragments. About 1 μg of fragment is isolated from the gel by electroelution. In the same manner as in Step 14, two oligodeoxynucleotides shown in Fig. 7, namely 5'-AATTCATGTCATCTTCTCGAACC-3' and 5'-TCGGGGTTCGAGAAGATGACATG-3' are synthesized. Then, each 5' end of the two oligodeoxynucleotides (about 100 pmole) is phosphorylated using T4 polynucleotide kinase in accordance with the method described in Literature (3), page 122. After completion of the reaction, the reaction mixture is extracted with phenol and then with chloroform. Then the so-obtained synthetic oligomers are mixed with 0.5 μg of the previously obtained *Ava*I-*Eco*RI fragment from

plasmid pHGEΔ3-1 and ethanol precipitated. These fragments are ligated at 4°C overnight using 10 units of T4 ligase in accordance with the procedure described in Literature (1), page 37. After completion of the reaction, the mixture is ethanol precipitated, followed by electrophoresis effected on a 4% by weight polyacrylamide gel to recover fragment by electroelution.

In accordance with the procedure described in F. Fuller, "Gene", *19*, pp. 42-54 (1982), plasmid pOP95-15 is prepared.

One $\mu$g of pOP 95-15 is digested with *Eco*RI and extracted with phenol and then with chloroform, followed by ethanol precipitation to obtain a vector. Using T4 DNA ligase, 0.5 $\mu$g of the obtained vector is ligated with the above-obtained fragment. In accordance with the procedure described in Literature (4), page 20, *E. coli* JM101 (ATCC 33876) is transformed using the above-obtained vector and cultivated on an agar medium containing 1 mM of IPTG and 0.004 w/v % x-gal to obtain about 100 white colonies.

Plasmid DNA is prepared from these transformants and digested with *Eco*RI to identify those plasmids containing the intended *Eco*Ri fragment. In order to examine the direction of insertion, those plasmids are digested with *Pvu*II and *Pst*I and electrophoresed on a 1.5% by weight agarose gel to select plasmids yielding fragments of about 1280 base pairs and about 2600 base pairs indicating that the direction of transcription of the *lac* UV5 promoter is in agreement with those of the oligodeoxynucleotides coding for TNF.

Base sequence analysis shows that these 2 plasmids have the same sequence and that the *lac* UV5 promoter, the synthesized oligodeoxynucleotide and DNA are properly combined with each other. The obtained plasmids is designated pHTNF-lacUV5-2.

*E. coli* containing pHTNF-lacUV5-2 is cultured in a conventional nutrient medium. Bioassay of the product for TNF activity indicates almost the same activity which is obtained with a plasmid pTNF-lacUV5-1 containing the rabbit TNF gene under control of the *lac* promoter.

Referential Example 3

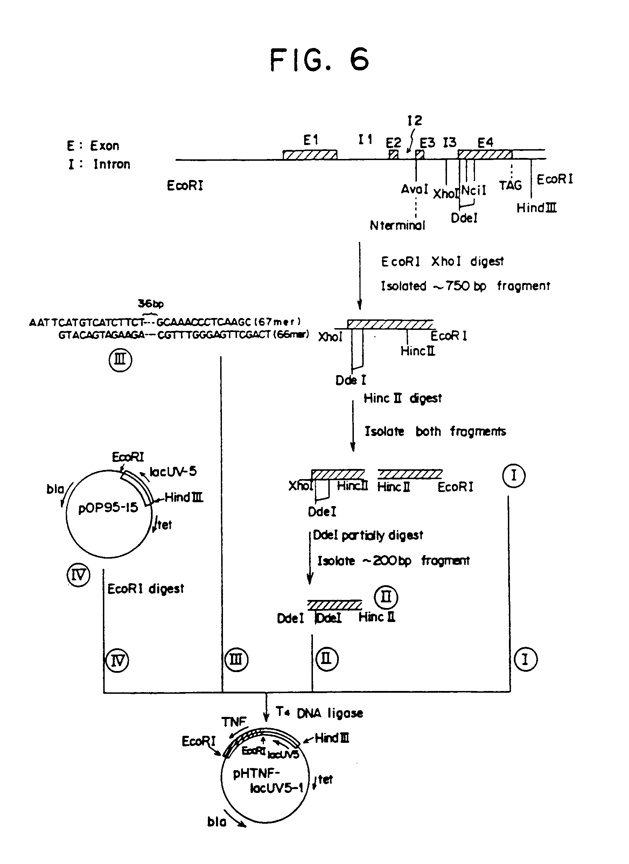

Using the plasmid pHGE and oligodeoxynucleotides 1 to 4 obtained by the procedure described in Steps 1 to 14 of Referential Example 2, pHTNF-lacUV5-1 is prepared in accordance with the procedure illustrated in Fig. 6.

Example 1

*E. coli* containing pHTNF-lacUV5-2 prepared in Referential Example 3 is cultured in a conventional nutrient medium and subjected to induction according to a customary method. The *E. coli* is further cultured to obtain cells of *E. coli* containing the physiologically active substance to be used in the present invention. The cells are collected by centrifugation and subjected to cell lysis by sonication in 1 l of 0.04 M Tris-HCl buffer (pH 7.8) to obtain a cell extract containing the physiologically active substance. The cell extract exhibits a cytotoxic activity of 4.5 $\times$ $10^5$ U/ml. A specific activity of the active substance in the extract is 3.0 $\times$ $10^4$ U/mg-protein.

Then, the extract is applied to a DEAE-Sepharose CL-6B (manufactured by Pharmacia Fine Chemicals AB, Sweden) column sufficiently equilibrated with 0.04 M Tris-HCl buffer (pH 8.0). The column is washed with 0.04 M Tris-HCl buffer (pH 8.0) and, thereafter, the elution is effected using 0.04 M Tris-HCl buffer (pH 8.0) containing 0.1 M NaCl. Active fractions are pooled and concentrated by ultrafiltration, thereby to obtain a crude solution containing the active substance having a specific activity of 4.0 $\times$ $10^5$ U/mg-protein.

The thus obtained crude solution is applied on a Sephacryl S-200 (manufactured by Pharmacia Fine Chemicals AB, Sweden) column equilibrated with 5 mM phosphate buffer (pH 7.4) containing 0.15 M NaCl, followed by gel filtration using the same buffer. Active fractions are pooled and concentrated by ultra-filtration, thereby to obtain a solution containing the physiologically active substance having a specific activity of 7.0 $\times$ $10^5$ U/mg-protein. An aliquot of the obtained solution is diluted with 5 mM phosphate buffer (pH 7.4) containing 0.15 M NaCl to prepare a solution of the physiologically active substance having a cytoxic activity of 120 U/ml.

To aliquots of the thus obtained solution is separately added each of HSA (human serum albumin) and BSA (Bovine serum albumin) to form sample solutions having albumin concentrations as indicated in Table 5. For each of the resulting sample solutions, the remaining activity is determined with respect to the samples which are respectively subjected to storing for 2 days, 7 days and 30 days at 4°C. In carrying out the above test, the active substance solution in which no albumin is incorporated is used as control.

To determine the remaining activity, the activity of each sample is assayed in vitro or in vivo, according to the methods as described hereinbefore. In the in vitro method, the remaining activity (%) is calculated from the assay value according to the following equation:

$$\text{Remaining activity (\%)} = \frac{A}{B} \times 100$$

(wherein A is the cytotoxic activity of the sample after storing or physical treatment and B is the cytotoxic activity of the sample before storing or physical treatment)

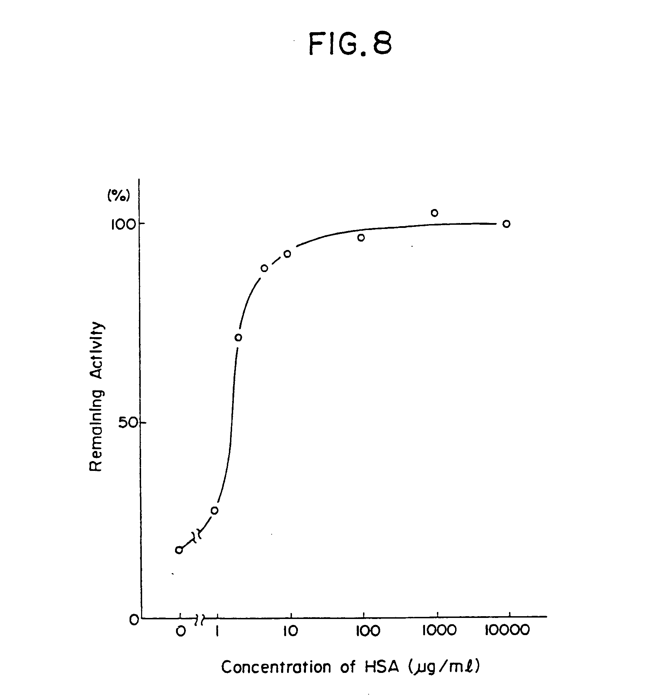

In the in vivo method, each sample solution is concentrated to have a concentration 20 times that at start by means of the Mini-Module NM-3 (trade mark of the ultra-filtration equipment manufactured by Asahi Chemical Industry Co. Ltd., Japan and sold by Funakoshi Yakuhin, Japan). Then, 0.5 ml of each of the thus concentrated TNF solutions is injected, through the tail vein, into each of a group of five tumor-bearing mice. The antitumor activity was assayed 24 hours later in accordance with the criterion as described hereinbefore. The results obtained are shown in Table 5 and Fig. 8.

Example 2

Substantially the same procedures as in example 1 are repeated to prepare sample solutions as described in Example 1.

For each of the solutions, the remaining activity is determined with respect to i) the samples respectively subjected to one cycle and three cycles of freezing (-70°C) and thawing and ii) the samples subjected to freezing at -70°C, lyophilization and storing at room temperature for 7 days. In carrying out the above test, the active substance solution containing no albumin is used as control. With respect to the lyophilized preparation [see ii) above], it is dissolved in sterile distilled water and then subjected to assay of the cytotoxic activity. The remaining activity of each sample is assayed in vivo or in vitro as in Example 1. The results are shown in Table 5.

Comparative Example

To aliquots of the human TNF solution having the same cytotoxic activity as that employed in Example 1, each of various amino acids, metal salts and chelating agents, which are well-known stabilizing agents for the solutions of ordinary physiologically active substances, is separately added in a varied concentration, as shown in Table 6. Each of the resulting solutions is stored at 4°C for 7 days, and subjected to the assay of the antitumor activity according to the in vivo assay method. The remaining activity (%) is calculated in the same manner as in Example 1. The results are shown in Table 6.

## Table 5

| Condition | | Example 1 | | | | Example 2 | |
|---|---|---|---|---|---|---|---|
| | | Storing at 4°C (Solution) | | Storing at 4°C (Solution) | | Freezing (-70°C) and thawing | Storing at room temperature (lyophilized preparation) |
| Assay method of activity | | in vitro | | in vivo | | in vitro | in vitro | in vivo |
| Stabilizing agent | Concentration mg/ml | days | | days | | repetition | days | days |
| | | 0 | 2 | 7 | 30 | 0 | 7 | 1 | 3 | 7 | 7 |
| None (Control) | — | 100 | 45 | 14 | 2 | +++3,++2 | +2, -3 | 40 | 14 | 35 | +4, -1 |
| HSA | 0.1 | 100 | 99 | 95 | 91 | ditto | +++3,++2 | 95 | 85 | 92 | +++3, ++2 |
| HSA | 1 | 100 | 98 | 96 | 90 | ditto | +++3,++2 | 99 | 95 | 101 | ++++4, +1 |
| BSA | 1 | 100 | 97 | 98 | 94 | ditto | +++4,++1 | 100 | 95 | 97 | +++3, ++2 |

Note: The figures in the columns marked "in vitro" represent the remaining activity as defined hereinbefore.

The figures in the columns marked "in vivo" represent the number of mice.
The meaning of symbols (-, +, ++, etc.) is given hereinbefore.

HSA : product of Sigma Chemical Co., U.S.A.
BSA : product of Armour Pharmaceutical Co., U.S.A.

EP 0 166 996 B2

TABLE 6

| Stabilizing agent | Concentration | Remaining activity, %<br>Storing at 4°C for 7 days |
|---|---|---|
| None (Control) | - | 14 |
| HSA | 1 mg/ml | 96 |
| Glycine | 0.1 M | 13 |
| L-Lysine | 0.1 M | 10 |
| L-Arginine | 0.1 M | 17 |
| L-Glutaric acid | 0.1 M | 15 |
| $CaCl_2$ | 1 mM | 14 |
| $MgCl_2$ | 1 mM | 18 |
| EDTA | 1 mM | 12 |

**Claims**

1.  A stable aqueous solution or powder containing a physiologically active substance and an effective amount of albumin, said physiologically active substance being one which is produced by recombinant DNA technique using a recombinant DNA containing a DNA coding for the physiologically active substance, said physiologically active substance being a polypeptide having an amino acid sequence represented by the following formula (I):

```
Ser Ser Ser Arg Thr Pro Ser Asp Lys Pro Val Ala His Val Val

Ala Asn Pro Gln Ala Glu Gly Gln Leu Gln Trp Leu Asn Arg Arg

Ala Asn Ala Leu Leu Ala Asn Gly Val Glu Leu Arg Asp Asn Gln

Leu Val Val Pro Ser Glu Gly Leu Tyr Leu Ile Tyr Ser Gln Val

Leu Phe Lys Gly Gln Gly Cys Pro Ser Thr His Val Leu Leu Thr

His Thr Ile Ser Arg Ile Ala Val Ser Tyr Gln Thr Lys Val Asn

Leu Leu Ser Ala Ile Lys Ser Pro Cys Gln Arg Glu Thr Pro Glu

Gly Ala Glu Ala Lys Pro Trp Tyr Glu Pro Ile Tyr Leu Gly Gly

Val Phe Gln Leu Glu Lys Gly Asp Arg Leu Ser Ala Glu Ile Asn

Arg Pro Asp Tyr Leu Asp Phe Ala Glu Ser Gly Gln Val Tyr Phe

Gly Ile Ile Ala Leu
```

wherein Gln stands for a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tryptophan residue, Arg an arginine residue, Met a methionine residue, and Cys a cysteine residue.

2. A stable aqueous solution or powder according to claim 1, wherein said DNA coding for the physiologically active substance comprises at least one base sequence selected from the group consisting of a base sequence represented by the following formula (II) and a complementary base sequence to said base sequence:

```
TCA TCT TCT CGA ACC CCG AGT GAC AAG CCT GTA GCC CAT GTT GTA

GCA AAC CCT CAA GCT GAG GGG CAG CTC CAG TGG CTG AAC CGC CGG

GCC AAT GCC CTC CTG GCC AAT GGC GTG GAG CTG AGA GAT AAC CAG

CTG GTG GTG CCA TCA GAG GGC CTG TAC CTC ATC TAC TCC CAG GTC

CTC TTC AAG GGC CAA GGC TGC CCC TCC ACC CAT GTG CTC CTC ACC

CAC ACC ATC AGC CGC ATC GCC GTC TCC TAC CAG ACC AAG GTC AAC

CTC CTC TCT GCC ATC AAG AGC CCC TGC CAG AGG GAG ACC CCA GAG

GGG GCT GAG GCC AAG CCC TGG TAT GAG CCC ATC TAT CTG GGA GGG

GTC TTC CAG CTG GAG AAG GGT GAC CGA CTC AGC GCT GAG ATC AAT

CGG CCC GAC TAT CTC GAC TTT GCC GAG TCT GGG CAG GTC TAC TTT

GGG ATC ATT GCC CTG
```

wherein A stands for a deoxyadenylic acid residue, G a deoxyguanylic acid residue, C a deoxycytidylic acid residue and T a thymidylic acid residue and wherein the left end and right end of the formula (II) represent 5'-hydroxyl group side and 3'-hydroxyl group side, respectively.

3. A stable aqueous solution or powder according to cam 1, wherein said albumin is human serum albumin.

4. A stable aqueous solution according to any one of claims 1 to 3, wherein said albumin is contained in the aqueous solution in an amount of about 10 $\mu$g to 50 mg per ml of the aqueous solution having a cytotoxic activity against L-M cells of $10^2$ to $10^9$ units/ml.

5. A stable aqueous solution according to claim 4, wherein said amount of an albumin is about 100 $\mu$g to 10 mg per ml of the aqueous solution.

6. A stable powder according to any one of claims 1 to 3, wherein said albumin is contained in the powder in such an amount as will, when the powder containing the physiologically active substance is dissolved in water to obtain an aqueous solution exhibiting a cytotoxic activity of $10^2$ to $10^9$ units/ml, give a concentration of about 10 $\mu$g to 50 mg per ml of the aqueous solution.

7. A stable powder according to claim 6, said concentration of an albumin is about 100 $\mu$g to 10 mg per ml of the aqueous solution.

8. A method for stabilizing a physiologically active substance, which comprises adding to an aqueous solution or powder containing a physiologically active substance an effective amount of an albumin, said physiologically active substance being one which is produced by recombinant DNA technique using a recombinant DNA containing a DNA coding for the physiologically active substance, said phyiologically active substance being a polypeptide having an amino acid sequence represented by the following formula (I):

33

Ser Ser Ser Arg Thr Pro Ser Asp Lys Pro Val Ala His Val Val

Ala Asn Pro Gln Ala Glu Gly Gln Leu Gln Trp Leu Asn Arg Arg

Ala Asn Ala Leu Leu Ala Asn Gly Val Glu Leu Arg Asp Asn Gln

Leu Val Val Pro Ser Glu Gly Leu Tyr Leu Ile Tyr Ser Gln Val

Leu Phe Lys Gly Gln Gly Cys Pro Ser Thr His Val Leu Leu Thr

His Thr Ile Ser Arg Ile Ala Val Ser Tyr Gln Thr Lys Val Asn

Leu Leu Ser Ala Ile Lys Ser Pro Cys Gln Arg Glu Thr Pro Glu

Gly Ala Glu Ala Lys Pro Trp Tyr Glu Pro Ile Tyr Leu Gly Gly

Val Phe Gln Leu Glu Lys Gly Asp Arg Leu Ser Ala Glu Ile Asn

Arg Pro Asp Tyr Leu Asp Phe Ala Glu Ser Gly Gln Val Tyr Phe

Gly Ile Ile Ala Leu

wherein Gln stands for a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tryptophan residue, Arg an arginine residue, Met a methionine residue, and Cys a cysteine residue.

9. A method according to claim 8, wherein said DNA coding for the physiologically active substance comprises at least one base sequence selected from the group consisting of a base sequence respresented by the following formula (II) and a complementary base sequence to said base sequence:

TCA TCT TCT CGA ACC CCG AGT GAC AAG CCT GTA GCC CAT GTT GTA

GCA AAC CCT CAA GCT GAG GGG CAG CTC CAG TGG CTG AAC CGC CGG

GCC AAT GCC CTC CTG GCC AAT GGC GTG GAG CTG AGA GAT AAC CAG

CTG GTG GTG CCA TCA GAG GGC CTG TAC CTC ATC TAC TCC CAG GTC

CTC TTC AAG GGC CAA GGC TGC CCC TCC ACC CAT GTG CTC CTC ACC

CAC ACC ATC AGC CGC ATC GCC GTC TCC TAC CAG ACC AAG GTC AAC

CTC CTC TCT GCC ATC AAG AGC CCC TGC CAG AGG GAG ACC CCA GAG

GGG GCT GAG GCC AAG CCC TGG TAT GAG CCC ATC TAT CTG GGA GGG

GTC TTC CAG CTG GAG AAG GGT GAC CGA CTC AGC GCT GAG ATC AAT

CGG CCC GAC TAT CTC GAC TTT GCC GAG TCT GGG CAG GTC TAC TTT

GGG ATC ATT GCC CTG

wherein A stands for a deoxyadenylic acid residue, G a deoxyguanylic acid residue, C a deoxycytidylic acid residue and T a thymidyiic acid residue and wherein the left end and right end of the formula (II) represent 5'-hydroxyl group side and 3'-hydroxyl group side, respectively.

**10.** A method according to claim 8, wherein said albumin is human serum albumin.

**11.** A method according to any one of claims 8 to 10, wherein said albumin is added to the aqueous solution in an amount of about 10 $\mu$g to 50 mg per ml of the aqueous solution having a cytotoxic activity against L-M cells of $10^2$ to $10^9$ units/ml.

**12.** A method according to claim 11, wherein said amount of an albumin is about 100 $\mu$g to 10 mg per ml of the aqueous solution.

**13.** A method according to any one of claims 8 to 10, wherein said albumin is added to the powder in such an amount as will, when the powder containing the physiologically active substance is dissolved in water to obtain an aqueous solution exhibiting a cytotoxic activity of $10^2$ to $10^9$ units/ml, give a concentration of about 10 $\mu$g to 50 mg per ml of the aqueous solution.

**14.** A method according to claim 13, wherein said concentration of an albumin is about 100 $\mu$g to 10 mg per ml of the aqueous solution.

**15.** A method according to any one of claims 8 to 12, which further comprises subjecting to lyophilization the resulting aqueous solution having said albumin added thereto.

**Patentansprüche**

**1.** Stabile wäßrige Lösung oder Pulver mit einem Gehalt an einer physiologisch wirksamen Substanz und einer wirksamen Menge an Albumin, worin die physiologisch wirksame Substanz eine Substanz ist, die durch rekombinante DNA-Technik unter Verwendung rekombinanter DNA mit einer DNA hergestellt wird, die die physiologisch wirksame Substanz kodiert, wobei die physiologisch wirksame Substanz ein Polypeptid ist, das eine Aminosäuresequenz der folgenden Formel (I) besitzt:

```
Ser Ser Ser Arg Thr Pro Ser Asp Lys Pro Val Ala His Val Val

Ala Asn Pro Gln Ala Glu Gly Gln Leu Gln Trp Leu Asn Arg Arg

Ala Asn Ala Leu Leu Ala Asn Gly Val Glu Leu Arg Asp Asn Gln

Leu Val Val Pro Ser Glu Gly Leu Tyr Leu Ile Tyr Ser Gln Val

Leu Phe Lys Gly Gln Gly Cys Pro Ser Thr His Val Leu Leu Thr

His Thr Ile Ser Arg Ile Ala Val Ser Tyr Gln Thr Lys Val Asn

Leu Leu Ser Ala Ile Lys Ser Pro Cys Gln Arg Glu Thr Pro Glu

Gly Ala Glu Ala Lys Pro Trp Tyr Glu Pro Ile Tyr Leu Gly Gly

Val Phe Gln Leu Glu Lys Gly Asp Arg Leu Ser Ala Glu Ile Asn

Arg Pro Asp Tyr Leu Asp Phe Ala Glu Ser Gly Gln Val Tyr Phe

Gly Ile Ile Ala Leu
```

worin Gln einen Glutaminrest, Asp einen Asparaginsäurerest, Pro einen Prolinrest, Tyr einen Tyrosinrest, Val einen Valinrest, Lys einen Lysinrest, Glu einen Glutaminsäurerest, Ala einen Alaninrest, Asn einen Asparaginrest, Leu einen Leucinrest, Phe einen Phenylalaninrest, Gly einen Glycinrest, His einen Hisdinrest, Ser einen Serinrest, Thr einen Threoninrest, Ile einen Isoleucinrest, Trp eine Tryptophanrest, Arg einen Argininrest, Met einen Methioninrest und Cys einen Cysteinrest bedeuten.

**2.** Stabile wäßrige Lösung oder Pulver nach Anspruch 1, wobei die die physiologisch wirksame Substanz kodierende DNA mindestens eine Basensequenz umfaßt, die aus der eine Basensequenz der folgenden Formel (II) und eine zu dieser Basensequenz komplementäre Basensequenz umfassende Gruppe ausgewählt ist:

TCA TCT TCT CGA ACC CCG AGT GAC AAG CCT GTA GCC CAT GTT GTA

GCA AAC CCT CAA GCT GAG GGG CAG CTC CAG TGG CTG AAC CGC CGG

GCC AAT GCC CTC CTG GCC AAT GGC GTG GAG CTG AGA GAT AAC CAG

CTG GTG GTG CCA TCA GAG GGC CTG TAC CTC ATC TAC TCC CAG GTC

CTC TTC AAG GGC CAA GGC TGC CCC TCC ACC CAT GTG CTC CTC ACC

CAC ACC ATC AGC CGC ATC GCC GTC TCC TAC CAG ACC AAG GTC AAC

CTC CTC TCT GCC ATC AAG AGC CCC TGC CAG AGG GAG ACC CCA GAG

GGG GCT GAG GCC AAG CCC TGG TAT GAG CCC ATC TAT CTG GGA GGG

GTC TTC CAG CTG GAG AAG GGT GAC CGA CTC AGC GCT GAG ATC AAT

CGG CCC GAC TAT CTC GAC TTT GCC GAG TCT GGG CAG GTC TAC TTT

GGG ATC ATT GCC CTG

worin A einen Deoxyadenylsäurerest, G einen Deoxyguanylsäurerest, C einen Deoxycytidylsäurerest und T einen Thymidylsäurerest bedeuten und worin das linke Ende und das rechte Ende der Formel (II) die Seite der 5'-Hydroxylgruppe bzw. die Seite der 3'-Hydroxylgruppe bezeichnen.

**3.** Stabile wäßrige Lösung oder Pulver nach Anspruch 1, worin das Albumin Humanserumalbumin ist.

**4.** Stabile wäßrige Lösung nach einem der Ansprüche 1 bis 3, worin das Albumin in der wäßrigen Lösung in einer Menge von etwa 10 $\mu$g bis 50 mg pro ml der wäßrigen Lösung mit einer cytotoxischen Aktivität gegen L-M Zellen von $10^2$ bis $10^9$ Einheiten/ml enthalten ist.

**5.** Stabile wäßrige Lösung nach Anspruch 4, worin die Menge an Albumin etwa 100 $\mu$g bis 10 mg pro ml der wäßrigen Lösung beträgt.

**6.** Stabiles Pulver nach einem der Ansprüche 1 bis 3, worin das Albumin in dem Pulver in einer derartigen Menge enthalten ist, daß sich dann, wenn das Pulver mit einem Gehalt an der physiologisch wirksamen Substanz in Wasser zur Herstellung einer wäßrigen Lösung mit einer cytotoxischen Aktivität von $10^2$ bis $10^9$ Einheiten/ml gelöst wird, sich eine Konzentration von etwa 10 $\mu$g bis 50 mg pro ml der wäßrigen Lösung ergibt.

**7.** Stabiles Pulver nach Anspruch 6, worin die Konzentration an Albumin etwa 100 $\mu$g bis 10 mg pro ml der wäßrigen Lösung beträgt.

**8.** Verfahren zum Stabilisieren einer physiologisch wirksamen Substanz, bei dem man zu einer wäßrigen Lösung oder einem Pulver mit einem Gehalt an physiologisch wirksamer Substanz eine wirksame Menge an Albumin zugibt, bei dem es sich bei der physiologisch wirksamen Substanz um eine Substanz handelt, die durch rekombinante DNA-Technik unter Verwendung rekombinanter DNA mit einem Gehalt an DNA hergestellt wird, die für die physiologisch wirksame Substanz kodiert, wobei die physiologisch wirksame Substanz ein Polypeptid mit einer Aminosäuresequenz der folgenden Formel

(I) ist:

```
Ser Ser Ser Arg Thr Pro Ser Asp Lys Pro Val Ala His Val Val

Ala Asn Pro Gln Ala Glu Gly Gln Leu Gln Trp Leu Asn Arg Arg

Ala Asn Ala Leu Leu Ala Asn Gly Val Glu Leu Arg Asp Asn Gln

Leu Val Val Pro Ser Glu Gly Leu Tyr Leu Ile Tyr Ser Gln Val

Leu Phe Lys Gly Gln Gly Cys Pro Ser Thr His Val Leu Leu Thr

His Thr Ile Ser Arg Ile Ala Val Ser Tyr Gln Thr Lys Val Asn

Leu Leu Ser Ala Ile Lys Ser Pro Cys Gln Arg Glu Thr Pro Glu

Gly Ala Glu Ala Lys Pro Trp Tyr Glu Pro Ile Tyr Leu Gly Gly

Val Phe Gln Leu Glu Lys Gly Asp Arg Leu Ser Ala Glu Ile Asn

Arg Pro Asp Tyr Leu Asp Phe Ala Glu Ser Gly Gln Val Tyr Phe

Gly Ile Ile Ala Leu
```

worin Gln einen Glutaminrest, Asp einen Asparaginsäurerest, Pro einen Prolinrest, Tyr einen Tyrosinrest, Val einen Valinrest, Lys einen Lysinrest, Glu einen Glutaminsäurerest, Ala einen Alaninrest, Asn einen Asparaginrest, Leu einen Leucinrest, Phe einen Phenylalaninrest, Gly einen Glycinrest, His einen Histidinrest, Ser einen Serinrest, Thr einen Threoninrest, Ile einen Isoleucinrest, Trp einen Tryptophanrest, Arg einen Argininrest, Met einen Methioninrest und Cys einen Cysteinrest bedeuten.

9.  Verfahren nach Anspruch 8, wobei die DNA, die für die physiologisch wirksame Substanz kodiert, mindestens eine Basensequenz umfaßt, die aus der Gruppe der Basensequenz der folgenden Formel (II) und einer zu dieser Basensequenz komplementären Basensequenz ausgewählt ist:

```
TCA TCT TCT CGA ACC CCG AGT GAC AAG CCT GTA GCC CAT GTT GTA

GCA AAC CCT CAA GCT GAG GGG CAG CTC CAG TGG CTG AAC CGC CGG

GCC AAT GCC CTC CTG GCC AAT GGC GTG GAG CTG AGA GAT AAC CAG

CTG GTG GTG CCA TCA GAG GGC CTG TAC CTC ATC TAC TCC CAG GTC

CTC TTC AAG GGC CAA GGC TGC CCC TCC ACC CAT GTG CTC CTC ACC

CAC ACC ATC AGC CGC ATC GCC GTC TCC TAC CAG ACC AAG GTC AAC

CTC CTC TCT GCC ATC AAG AGC CCC TGC CAG AGG GAG ACC CCA GAG

GGG GCT GAG GCC AAG CCC TGG TAT GAG CCC ATC TAT CTG GGA GGG

GTC TTC CAG CTG GAG AAG GGT GAC CGA CTC AGC GCT GAG ATC AAT

CGG CCC GAC TAT CTC GAC TTT GCC GAG TCT GGG CAG GTC TAC TTT

GGG ATC ATT GCC CTG
```

worin A einen Deoxyadenylsäurerest, G einen Deoxyguanylsäurerest, C einen Deoxycytidylsäurerest und T einen Thymidylsäurerest bedeuten und worin das linke Ende und das rechte Ende der Formel (II) die Seite der 5'-Hydroxylgruppe bzw. die Seite der 3'-Hydroxylgruppe bezeichnen.

**10.** Verfahren nach Anspruch 8, bei dem das Albumin Humanserumalbumin ist.

**11.** Verfahren nach einem der Ansprüche 8 bis 10, bei dem das Albumin zu der wäßrigen Lösung in einer Menge von etwa 10 μg bis 50 mg pro ml der wäßrigen Lösung mit einer cytotoxischen Aktivität gegen L-M-Zellen von $10^2$ bis $10^9$ Einheiten/ml zugegeben wird.

**12.** Verfahren nach Anspruch 11, bei dem die Menge an Albumin etwa 100 μg bis 10 mg pro ml der wäßrigen Lösung beträgt.

**13.** Verfahren nach einem der Ansprüche 8 bis 10, bei dem das Albumin zum Pulver in einer derartigen Menge zugegeben wird, daß sich dann, wenn das Pulver mit einem Gehalt an der physiologisch wirksamen Substanz in Wasser zur Herstellung einer wäßrigen Lösung mit einer cytotoxischen Aktivität von $10^2$ bis $10^9$ Einheiten/ml gelöst wird, eine Konzentration von etwa 10 μg bis 50 mg pro ml der wäßrigen Lösung ergibt.

**14.** Verfahren nach Anspruch 13, bei dem die Konzentration an Albumin etwa 100 μg bis 10 mg pro ml der wäßrigen Lösung beträgt.

**15.** Verfahren nach einem der Ansprüche 8 bis 12, bei dem man ferner die resultierende wäßrige Lösung mit zugegebenem Albumin einer Lyophilisation unterwirft.

**Revendications**

**1.** Solution aqueuse stable ou poudre contenant une substance physiologiquement active et une quantité efficace d'albumine, ladite substance active physioloqiquement étant une substance qui est produite par une technique d'ADN recombinant contenant un ADN codant pour la substance active physiologiquement, ladite substance active physioloqiquement étant un polypeptide ayant une séquence acide aminé représentée par la formule suivante (I) :

```
Ser Ser Ser Arg Thr Pro Ser Asp Lys Pro Val Ala His Val Val
Ala Asn Pro Gln Ala Glu Gly Gln Leu Gln Trp Leu Asn Arg Arg
Ala Asn Ala Leu Leu Ala Asn Gly Val Glu Leu Arg Asp Asn Gln
Leu Val Val Pro Ser Glu Gly Leu Tyr Leu Ile Tyr Ser Gln Val
Leu Phe Lys Gly Gln Gly Cys Pro Ser Thr His Val Leu Leu Thr
His Thr Ile Ser Arg Ile Ala Val Ser Tyr Gln Thr Lys Val Asn
Leu Leu Ser Ala Ile Lys Ser Pro Cys Gln Arg Glu Thr Pro Glu
Gly Ala Glu Ala Lys Pro Trp Tyr Glu Pro Ile Tyr Leu Gly Gly
Val Phe Gln Leu Glu Lys Gly Asp Arg Leu Ser Ala Glu Ile Asn
Arg Pro Asp Tyr Leu Asp Phe Ala Glu Ser Gly Gln Val Tyr Phe
Gly Ile Ile Ala Leu
```

dans laquelle Gln représente un résidu glutamine, Asp un résidu acide aspartique, Pro un résidu proline, Tyr un résidu tyrosine, Val un résidu valine, Lys un résidu lysine, Glu un résidu acide glutamique, Ala un résidu alanine, Asn un résidu asparaqine, Leu un résidu leucine, Phe un résidu phénylalanine, Gly un résidu glycine, His un résidu histidine, Ser un résidu sérine, Thr un résidu thréonine, Ile un résidu isoleucine, Trp un résidu tryptophane, Arg un résidu arginine, Met un résidu méthionine et Cys un résidu cystéine.

**2.** Solution aqueuse stable ou poudre selon la revendication 1, dans laquelle ledit ADN codant pour la substance physiologiquement active comprend au moins une séquence de base choisie dans le groupe consistant d'une séquence de base représentée par la formule (II) suivante et une séquence de base complémentaire à ladite séquence de base :

```
TCA TCT TCT CGA ACC CCG AGT GAC AAG CCT GTA GCC CAT GTT GTA
GCA AAC CCT CAA GCT GAG GGG CAG CTC CAG TGG CTG AAC CGC CGG
GCC AAT GCC CTC CTG GCC AAT GGC GTG GAG CTG AGA GAT AAC CAG
CTG GTG GTG CCA TCA GAG GGC CTG TAC CTC ATC TAC TCC CAG GTC
CTC TTC AAG GGC CAA GGC TGC CCC TCC ACC CAT GTG CTC CTC ACC
CAC ACC ATC AGC CGC ATC GCC GTC TCC TAC CAG ACC AAG GTC AAC
CTC CTC TCT GCC ATC AAG AGC CCC TGC CAG AGG GAG ACC CCA GAG
GGG GCT GAG GCC AAG CCC TGG TAT GAG CCC ATC TAT CTG GGA GGG
GTC TTC CAG CTG GAG AAG GGT GAC CGA CTC AGC GCT GAG ATC AAT
CGG CCC GAC TAT CTC GAC TTT GCC GAG TCT GGG CAG GTC TAC TTT
GGG ATC ATT GCC CTG
```

dans laquelle A représente un résidu acide désoxyadénylique, G un résidu acide désoxyguanylique,C un résidu acide désoxycytidylique et T un résidu acide thymidylique et dans lequel l'extrémité gauche et l'extrémité droite de la formule (II) représentent le côté du groupe 5'-hydroxyle et le côté du groupe 3'-hydroxyle, respectivement.

**3.** Solution aqueuse stable ou poudre selon la revendication 1, où ladite albumine est l'albumine de sérum humain.

**4.** Solution aqueuse stable selon l'une quelconque des revendications 1 à 3, où ladite albumine est contenue dans la solution aqueuse en une quantité d'environ 10 $\mu$g à 50 mg par ml de la solution aqueuse ayant une activité cytotoxique contre les cellules L-M de $10^2$ à $10^9$ unités/ml.

**5.** Solution aqueuse stable selon la revendication 4, où ladite quantité d'albumine est d'environ 100 $\mu$g à 10 mg par ml de la solution aqueuse.

**6.** Poudre stable selon l'une quelconque des revendications 1 à 3, où ladite albumine est contenue dans la poudre en une quantité telle que, quand la poudre contenant la substance physiologiquement active est dissoute dans l'eau pour obtenir une solution aqueuse présentant une activité cytotoxique de $10^2$ à $10^9$ unités par ml, cela donne une concentration d'environ 10 $\mu$g à 50 mg par ml de la solution aqueuse.

**7.** Poudre stable selon la revendication 6, ladite concentration d'albumine étant d'environ 100 $\mu$g à 10 mg par ml de la solution aqueuse.

**8.** Méthode pour stabiliser une substance physiologiquement active, qui comprend l'ajout, à une solution aqueuse ou une poudre contenant une substance physiologiquement active, d'une quantité efficace d'une albumine, ladite substance physiologiquement active étant une substance qui est produite par une technique d'ADN recombinant en utilisant un ADN recombinant contenant un ADN codant pour la substance physiologiquement active, ladite substance physiologiquement active étant un polypeptide ayant une séquence d'acides aminés représentée par la formule (I) suivante :

EP 0 166 996 B2

```
Ser Ser Ser Arg Thr Pro Ser Asp Lys Pro Val Ala His Val Val

Ala Asn Pro Gln Ala Glu Gly Gln Leu Gln Trp Leu Asn Arg Arg

Ala Asn Ala Leu Leu Ala Asn Gly Val Glu Leu Arg Asp Asn Gln

Leu Val Val Pro Ser Glu Gly Leu Tyr Leu Ile Tyr Ser Gln Val

Leu Phe Lys Gly Gln Gly Cys Pro Ser Thr His Val Leu Leu Thr

His Thr Ile Ser Arg Ile Ala Val Ser Tyr Gln Thr Lys Val Asn

Leu Leu Ser Ala Ile Lys Ser Pro Cys Gln Arg Glu Thr Pro Glu

Gly Ala Glu Ala Lys Pro Trp Tyr Glu Pro Ile Tyr Leu Gly Gly

Val Phe Gln Leu Glu Lys Gly Asp Arg Leu Ser Ala Glu Ile Asn

Arg Pro Asp Tyr Leu Asp Phe Ala Glu Ser Gly Gln Val Tyr Phe

Gly Ile Ile Ala Leu
```

dans laquelle Gln représente un résidu glutamine, Asp un résidu acide aspartique, Pro un résidu proline, Tyr un résidu tyrosine, Val un résidu valine, Lys un résidu lysine, Glu un résidu acide glutamique, Ala un résidu alanine, Asp un résidu asparagine, Leu un résidu leucine, Phe un résidu phénylalanine, Gly un résidu glycine, His un résidu histidine Ser un résidu sérine, Thr un résidu thréonine, Ile un résidu isoleucine, Trp un résidu tryptophane, Arg un résidu arginine, Met un résidu méthionine et Cys un résidu cystéine.

9.  Méthode selon la revendication 8, dans laquelle ledit ADN codant pour la substance physiologiquement active comprend au moins une séquence de base choisie dans le groupe consistant d'une séquence de base représentée par la formule (II) suivante et une séquence de base complémentaire à ladite séquence de base :

```
TCA TCT TCT CGA ACC CCG AGT GAC AAG CCT GTA GCC CAT GTT GTA

GCA AAC CCT CAA GCT GAG GGG CAG CTC CAG TGG CTG AAC CGC CGG

GCC AAT GCC CTC CTG GCC AAT GGC GTG GAG CTG AGA GAT AAC CAG

CTG GTG GTG CCA TCA GAG GGC CTG TAC CTC ATC TAC TCC CAG GTC

CTC TTC AAG GGC CAA GGC TGC CCC TCC ACC CAT GTG CTC CTC ACC

CAC ACC ATC AGC CGC ATC GCC GTC TCC TAC CAG ACC AAG GTC AAC

CTC CTC TCT GCC ATC AAG AGC CCC TGC CAG AGG GAG ACC CCA GAG

GGG GCT GAG GCC AAG CCC TGG TAT GAG CCC ATC TAT CTG GGA GGG

GTC TTC CAG CTG GAG AAG GGT GAC CGA CTC AGC GCT GAG ATC AAT

CGG CCC GAC TAT CTC GAC TTT GCC GAG TCT GGG CAG GTC TAT TTT

GGG ATC ATT GCC CTG
```

dans laquelle A représente un résidu acide désoxyadénylique, G un résidu acide désoxyguanylique, C un résidu acide désoxycytidylique et T un résidu acide thymidylique et dans laquelle l'extrémité droite et l'extrémité gauche de la formule (II) représentent un côté du groupe 5'-hydroxyle et un côté du groupe 3'-hydroxyle, respectivement.

10. Méthode selon la revendication 8, où ladite albumine est l'albumine de sérum humain.

11. Méthode selon l'une quelconque des revendications 8 à 10, où ladite albumine est ajoutée à une solution aqueuse en une quantité d'environ 10 $\mu$g à 50 mg par ml de la solution aqueuse ayant une activité cytotoxique contre les cellules L-M de $10^2$ à $10^9$ unités par ml.

40

**12.** Méthode selon la revendication 11, où ladite quantité d'albumine est d'environ 100 $\mu$g à 10 mg par ml de la solution aqueuse.

**13.** Méthode selon l'une quelconque des revendications 8 à 10, où ladite albumine est ajoutée à la poudre en une quantité telle que, quand la poudre contenant la substance physiologiquement active est dissoute dans l'eau pour obtenir une solution aqueuse présentant une activité cytotoxique de $10^2$ à $10^9$ unités/ml, cela donne une concentration d'environ 10 $\mu$g à 50 mg par ml de la solution aqueuse.

**14.** Méthode selon la revendication 13, où ladite concentration d'albumine est d'environ 100 $\mu$g à 10 mg par ml de la solution aqueuse.

**15.** Méthode selon l'une quelconque des revendications 8 à 12, qui consiste de plus à soumettre la solution aqueuse résultante, où est ajoutée ladite albumine, à une lyophilisation.

# FIG. I

FIG. 2

# FIG. 3

FIG.4

E : Exon
I : Intron

N-terminal of matured TNF

stop codon

# FIG. 5

E : Exon
I : Intron

# FIG. 6

# FIG. 7

Ava I

Ava I , EcoR I digest
Isolate fragment

AATTCATGTCATCTTCTCGAACC
GTACAGTAGAAGAGCTTGGGGCT

CCGAG ///// G
C ///// CTTAA

T4 DNA ligose
Isolate fragment

AATTCATGTCATCTTCTCGAACCCCGAG ///// G
GTACAGTAGAAGAGCTTGGGGCTC ///// CTTAA

EcoRI
lacUV5
bla
Hind III
pOP95-15
tet

EcoRI digest

T4 DNA ligase

EcoR I
TNF
lacUV5
EcoRI
Hind III
pHTNF
lacUV5-2
tet
Pst I
bla
Pvu II

# FIG.8